# EUROPÄISCHE PATENTSCHRIFT

(11) **EP 2 480 192 B1**
(45) Veröffentlichungstag und Bekanntmachung des Hinweises auf die Patenterteilung: **13.01.2016**
(21) Anmeldenummer: 10752835.8
(22) Anmeldetag: 15.09.2010
(51) Int. Cl.: A61K 8/26, A61K 8/39, A61Q 15/00, A61K 8/31, A61K 8/86, A61K 8/04

(54) **WASSERFREIE ANTITRANSPIRANT-SPRAYS MIT VERBESSERTER WIRKSTOFFFREISETZUNG**
NON-AEROSOL WATER-FREE ANTIPERSPIRANT IN WHICH ACTIVE SUBSTANCES ARE MORE READILY RELEASED
ANTI-TRANSPIRANTS NON AÉROSOL SANS EAU À LIBÉRATION AMÉLIORÉE D'AGENTS

(30) Priorität: 22.09.2009 DE 102009029669
(43) Veröffentlichungstag der Anmeldung: 01.08.2012
(73) Patentinhaber: Henkel AG & Co. KGaA, 40589 Düsseldorf (DE)
(72) Erfinder: BANOWSKI, Bernhard, 40597 Düsseldorf (DE); BUSE, Nadine, 40724 Hilden (DE)
(86) Internationale Anmeldenummer: PCT/EP2010/063568
(87) Internationale Veröffentlichungsnummer: WO 2011/036081

(56) Entgegenhaltungen:
- WO-A1-98/17238
- WO-A2-2009/083547
- DE-A1- 19 501 288
- US-A- 4 479 887
- US-A- 5 080 830
- US-A1- 2001 051 138

## Beschreibung

Gegenstand der vorliegenden Erfindung sind wasserfreie schweißhemmende Zusammensetzungen, insbesondere auf Basis einer wasserfreien, treibmittelfrei oder mit einem Treibmittel versprühbaren Suspension, die eine verbesserte Wirkstofffreisetzung des schweißhemmenden Wirkstoffs ermöglichen.

Wasserfreie, mit einem Treibmittel versprühbare Antitranspirant-Suspensionen enthalten neben schweißreduzierenden Wirkstoffen in der Regel mindestens ein kosmetisches Öl als Träger für den teilchenförmigen schweißreduzierenden Wirkstoff. Die Supensionen werden in einem druckfesten Behälter, meist einer Dose aus Weißblech oder Aluminium, die innen lackiert ist, zusammen mit einem verflüssigten Kohlenwasserstoff, wie n-Butan, iso-Butan und/oder Propan, als Treibmittel abgefüllt. Vor Gebrauch des Sprühventils, bei dem Treibmittel und eine Portion der Suspension freigesetzt werden, muss der Behälter zunächst ausreichend geschüttelt werden, um den abgesetzten Antitranspirantwirkstoff aufzumischen. Damit sich der suspendierte Antitranspirantwirkstoff nicht sofort wieder absetzt, enthalten handelsübliche Suspensionen ein Suspendiermittel, z. B. hydrophob modifizierte Hectorite, wie sie beispielsweise unter der Handelsbezeichnung Bentone Gel von den Firmen Rheox und Elementis Specialties erhältlich sind, oder auch hydrophile und/oder hydrophob modifizierte Kieselsäuren.

Bei den handelsüblichen Sprays ist der in dem wasserfreien Träger suspendierte Antitranspirantwirkstoff mit einer Ölschicht bedeckt. Während und nach der Applikation auf der Haut begünstigt diese Ölschicht das Sprühbild, das heißt, der Wirkstoff wird nicht zu sehr vernebelt, sondern gelangt gezielt auf die Haut; außerdem sorgt die Ölschicht für eine gewisse Haftung des pulverförmigen Antitranspirantwirkstoffs auf der Haut. Diese Ölschicht verzögert allerdings die Freisetzung des Antitranspirantwirkstoffs in die wirksame wasserlösliche Form.

Im Stand der Technik sind aus US 5118497 und US 5017360 wasserhaltige Antitranspirant-Cremes mit einem Wassergehalt von 43,9 Gew.-% bekannt, die mit Hilfe des Handelsprodukts Elfacos ST-37, einem Alkyl-modifizierten Polyether mit der INCI-Bezeichnung PEG-22/Dodecyl Glycol Copolymer, der einen HLB-Wert von 2,4 aufweist, emulgiert sind.

US 6613338 und insbesondere WO 98/17238 A1 offenbaren Stifte, insbesondere Antitranspirant-Stifte, in Form von festen Wasser-in-ÖI-Emulsionen mit einem Wassergehalt von 30 - 85 Gew.-%, die mit Hilfe eines Alkyl-modifizierten Polyethers der allgemeinen Formel ACTIVATOR-(I) mit der INCI-Bezeichnung Methoxy PEG-22/ Dodecyl Glycol Copolymer emulgiert sind. Dieser Stand der Technik gab dem Fachmann keinen Hinweis darauf, dass Alkyl-modifizierte Polyether der allgemeinen Formel ACTIVATOR-(I) die Freisetzung des Antitranspirantwirkstoffs aus einer wasserfreien Zusammensetzung verbessern können.

DE 19501288A1 offenbart Wasser-in-Öl-Emulsionen, in denen die vorliegend beanspruchten Alkyl-modifizierten Polyether der allgemeinen Formel ACTIVATOR-(I) einen Teil des W/O-Emulsionssystems darstellen.

US 5080830 beschäftigt sich gemäß der Zusammenfassung ebenfalls mit wässrigen und wasserbasierten Zusammensetzungen, die Methoxy PEG-17/Dodecyl Glycol Copolymer als Teil eines Stabilisator- und Dispergiermittelsystems umfasst und dabei insbesondere zur Formulierung von Haarconditionern und Wäscheweichspülmitteln geeignet ist.

US 2001/051138 A1 offenbart Antitranspirantzusammensetzungen in Form einer Wasser-in-Öl-Emulsion, die unter anderem 65 bis 75 Gew.-% einer wässrigen Lösung eines Antitranspirantwirkstoffs und 0,01 bis 8,5 Gew.-% eines Kupplungsmittels enthalten. Als geeignetes Kupplungsmittel ist unter anderem Methoxy PEG-22/Dodecyl Glycol Copolymer offenbart. Das Kupplungsmittel dient dazu, die Emulsion zu stabilisieren und Transparenz herzustellen.

WO 2009/083547 A2 und WO2009/083807 A2 lehren, dass bestimmte Organosiloxan-Oxyalkylen-Copolymere die Freisetzung des Antitranspirantwirkstoffs aus einer wasserfreien Zusammensetzung verbessern können. Als Nachteil dieser Substanzen hat sich aber herausgestellt, dass sie sich unter bestimmten Umständen, insbesondere wenn der kosmetische Träger wenig oder kein Siliconöl enthält, schlecht einarbeiten und homogen einmischen lassen.

Überraschend wurde nun gefunden, dass die Freisetzung des schweißhemmenden Wirkstoffs aus einer wasserfreien Antitranspirant-Zusammensetzung verbessert werden kann, wenn mindestens ein Alkyl-modifizierter Polyether der allgemeinen Formel ACTIVATOR-(I) 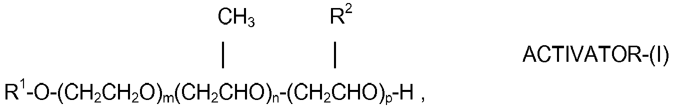
worin R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet, enthalten ist.

Gegenstand der vorliegenden Erfindung sind daher schweißhemmende Zusammensetzungen zur persönlichen Körperpflege, konfektioniert als treibmittelfrei oder mit einem Treibmittel versprühbare Suspension oder Lösung, enthaltend mindestens einen schweißhemmenden Wirkstoff, mindestens ein unter Normalbedingungen flüssiges Öl als Träger, 0 - 7 Gew.-%, bevorzugt 0 - 3 Gew.-%, freies Wasser, bezogen auf das Gewicht der treibmittelfreien Zusammensetzung, und mindestens einen Alkyl-modifizierten Polyether der allgemeinen Formel ACTIVATOR-(I) 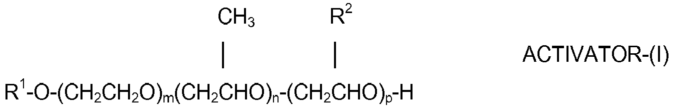
worin R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet.

Die erfindungsgemäßen schweißhemmenden Zusammensetzungen auf wasserfreier Basis sind als treibmittelfrei, beispielsweise in einem Pumpspender, oder mit einem Treibmittel versprühbare Suspension oder Lösung konfektioniert.

Die erfindungsgemäße schweißhemmende Wirkstoffkombination eignet sich bevorzugt für versprühbar konfektionierte Zusammensetzungen, insbesondere für treibmittelhaltige Suspensionen, die als Antitranspirant-Spray angewendet werden.

Alle im Folgenden gemachten Mengenangaben beziehen sich im Fall von Rezepturen, die mit einem Treibmittel versprüht werden (Aerosolsprays), auf das Gewicht der treibmittel-freien Zusammensetzung.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Erfindungsgemäß bevorzugte Zusammensetzungen sind dadurch gekennzeichnet, dass der Alkyl-modifizierte Polyether d) ausgewählt ist aus Verbindungen der allgemeinen Formel ACTIVATOR-(I), worin
R¹ ausgewählt ist aus einer Methylgruppe, einer Ethylgruppe, einer n-Propylgruppe und einer 1-Methylethylgruppe, bevorzugt einer Methylgruppe,
R² ausgewählt ist aus einer n-Octyl-, 2-Ethylhexyl-, n-Nonyl-, n-Decyl-, 2-Ethyloctyl-, n-Undecyl-, n-Dodecyl-, 2-Ethyldecyl-, n-Tridecyl-, Myristyl-, n-Pentadecyl-, Cetyl-, Palmityl-, Stearyl-, Elaidyl-, Arachidyl-, Behenyl- oder Cocyl-Gruppe, bevorzugt einer n-Decyl-Gruppe,
m eine rationale Zahl von 12 - 30, bevorzugt 22 - 23, darstellt,
n eine rationale Zahl von 0 - 8, bevorzugt 0 - 4, besonders bevorzugt 0, darstellt,
p eine rationale Zahl von 1 - 9, bevorzugt 4 - 8, besonders bevorzugt 5 - 7, darstellt.

Bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R¹ eine Methylgruppe darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R² eine n-Decyl-Gruppe darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen m eine rationale Zahl von 21 - 23 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen m eine rationale Zahl von 22 - 23 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen n = 0 ist.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen p eine rationale Zahl von 4 - 5 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen p eine rationale Zahl von 6 - 8 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen p eine rationale Zahl von 7 - 8 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R¹ eine Methylgruppe, R² eine n-Decyl-Gruppe, m eine rationale Zahl von 22 - 23, n = 0 und p eine rationale Zahl von 4 - 5 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R¹ eine Methylgruppe, R² eine n-Decyl-Gruppe, m eine rationale Zahl von 22 - 23, n = 0 und p eine rationale Zahl von 7 - 8 darstellt.

Weitere bevorzugte erfindungsgemäße Zusammensetzungen sind dadurch gekennzeichnet, dass der Alkyl-modifizierte Polyether d) der allgemeinen Formel ACTIVATOR-(I), worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10 und p eine rationale Zahl von 1 bis 10 ist, einen HLB-Wert im Bereich von 5 - 7, bevorzugt 6 - 6,8, besonders bevorzugt 6,2 - 6,5, aufweist.

Weitere bevorzugte erfindungsgemäße Zusammensetzungen sind dadurch gekennzeichnet, dass der Alkyl-modifizierte Polyether d) der allgemeinen Formel ACTIVATOR-(I), worin R¹ ausgewählt ist aus einer Methylgruppe, einer Ethylgruppe, einer n-Propylgruppe und einer 1-Methylethylgruppe, bevorzugt einer Methylgruppe, R² ausgewählt ist aus einer n-Octyl-, 2-Ethylhexyl-, n-Nonyl-, n-Decyl-, 2-Ethyloctyl-, n-Undecyl-, n-Dodecyl-, 2-Ethyldecyl-, n-Tridecyl-, Myristyl-, n-Pentadecyl-, Cetyl-, Palmityl-, Stearyl-, Elaidyl-, Arachidyl-, Behenyl- oder Cocyl-Gruppe, bevorzugt einer n-Decyl-Gruppe, m eine rationale Zahl von 12 - 30, bevorzugt 22 - 23, darstellt, n eine rationale Zahl von 0 - 8, bevorzugt 0 - 4, besonders bevorzugt 0, darstellt, p eine rationale Zahl von 1 - 9, bevorzugt 4 - 8, besonders bevorzugt 5 - 7, darstellt, einen HLB-Wert im Bereich von 5 - 7, bevorzugt 6 - 6,8, besonders bevorzugt 6,2 - 6,5, aufweist.

Weitere bevorzugte erfindungsgemäße Zusammensetzungen sind dadurch gekennzeichnet, dass der Alkyl-modifizierte Polyether d) der oben dargestellten allgemeinen Formel ACTIVATOR-(I), worin R¹ ausgewählt ist aus einer Methylgruppe, R² ausgewählt ist aus einer n-Decyl-Gruppe, m eine rationale Zahl von 22 - 23, n = 0 und p eine rationale Zahl von 4 - 8 ist, einen HLB-Wert im Bereich von 5 - 7, bevorzugt 6 - 6,8, besonders bevorzugt 6,2 - 6,5, aufweist.

Weitere bevorzugte erfindungsgemäße Zusammensetzungen sind dadurch gekennzeichnet, dass mindestens ein Alkyl-modifizierter Polyether der allgemeinen Formel ACTIVATOR-(I) enthalten ist mit einem HLB-Wert im Bereich von 5 - 7, bevorzugt 6 - 6,8, besonders bevorzugt 6,2 - 6,5, und mit R¹ = Methyl, R² = n-Decyl, m = 22, n = 0 und p = 4 - 5.

Weitere bevorzugte erfindungsgemäße Zusammensetzungen sind dadurch gekennzeichnet, dass mindestens ein Alkyl-modifizierter Polyether der allgemeinen Formel ACTIVATOR-(I) enthalten ist mit einem HLB-Wert im Bereich von 5 - 7, bevorzugt 6 - 6,8, besonders bevorzugt 6,2 - 6,5, und mit R¹ = Methyl, R² = n-Decyl, m = 22, n = 0 und p = 7 - 6.

Erfindungsgemäß bevorzugte Alkyl-modifizierte Polyether der allgemeinen Formel AKTIVATOR-(I) sind erhältlich gemäß dem in US 4479887, Beispiel 1, insbesondere Beispiel 1A und 1D, offenbarten Herstellungsverfahren aus einem C₁-C₃-Alkanolethoxylat und einem 1,2-Epoxy-C₁₀-C₃₂-Alkan. Erfindungsgemäß bevorzugte Zusammensetzungen sind dadurch gekennzeichnet, dass der Alkyl-modifizierte Polyether d) der allgemeinen Formel ACTIVATOR-(I) ausgewählt ist aus Verbindungen mit der INCI-Bozeichnung Methoxy PEG-22/Dodecyl Glycol Copolymer. Eine solche Verbindung ist beispielsweise als Handelsprodukt Elfacos E 200 erhältlich.

Weitere bevorzugte erfindungsgemäße Zusammensetzungen sind dadurch gekennzeichnet, dass mindestens ein Alkyl-modifizierter Polyether d) der oben dargestellten allgemeinen Formel ACTIVATOR-(I) in einer Gesamtmenge von 0,01 - 5 Gew.-%, bevorzugt 0,1 - 3 Gew.-%, besonders bevorzugt in einer Gesamtmenge von 0,5 - 2 Gew.-%o, außerordentlich bevorzugt in einer Gesamtmenge von 1 - 1,5 Gew.-%, enthalten ist, jeweils bezogen auf das Gesamtgewicht der treibmittelfreien Zusammensetzung.

Alkyl-modifizierte Polyether d) der oben dargestellten allgemeinen Formel ACTIVATOR-(I) lassen sich, auch wenn der kosmetische Träger wenig oder kein Siliconöl enthält, gut in die erfindungsgemäßen Zusammensetzungen einarbeiten und homogen einmischen.

Die erfindungsgemäßen Zusammensetzungen sind im Wesentlichen wasserfrei, d. h. sie enthalten D bis maximal 7 Gew.-%, bevorzugt 0 bis maximal 3 Gew.-% freies Wasser, außerordentlich bevorzugt 0 bis maximal 2 Gew.-% freies Wasser, jeweils bezogen auf die gesamte (treibmittelfreie) Zusammensetzung. Der Gehalt an Kristallwasser, Hydratationswasser oder ähnlich molekular gebundenem Wasser, der in den eingesetzten Bestandteilen, insbesondere in den schweißhemmenden Wirkstoffen enthalten sein kann, stellt im Sinne der vorliegenden Anmeldung kein freies Wasser dar.

In einer bevorzugten erfindungsgemäßen Ausführungsform sind die schweißhemmenden Wirkstoffe und gegebenenfalls weitere im Träger unlösliche Wirkstoffe in mindestens einem unter Normalbedingungen flüssigen Öl suspendiert. Zur besseren Anwendbarkeit wird dieser Suspension noch mindestens ein bevorzugt lipophiles Verdickungsmittel als Suspendierhilfe zugesetzt. Weitere bevorzugte erfindungsgemäße Zusammensetzungen sind daher dadurch gekennzeichnet, dass sie mindestens ein lipophiles Verdickungsmittel enthalten.

Bevorzugte erfindungsgemäße Zusammensetzungen sind dadurch gekennzeichnet, dass das mindestens eine lipophile Verdickungsmittel ausgewählt ist aus hydrophobierten Tonmineralien, pyrogenen Kieselsäuren, Bentone-Gelen, Ethylene/Propylene/Styrene Copolymeren, Butylene/ Ethylene/Styrene Copolymeren, Dextrinestern, Siliconelastomeren, unter Normalbedingungen festen Wachsen und/oder Glycerintriestern. Hierunter sind hydrophobierte Tonmineralien besonders bevorzugt. Die erfindungsgemäßen Zusammensetzungen enthalten in einer bevorzugten Ausführungsform mindestens ein Suspensions- oder Verdickungsmittel. Besonders geeignete Verdickungsmittel sind hydrophobierte Tonmineralien wie Montmorillonite, Hectorite und Bentonite, insbesondere Disteardimonium Hectorite und Quaternium-18 Hectorite. Die handelsüblichen Verdickungsmittel stellen diese hydrophobierten Tonmineralien als Pulver oder in Form eines vorgefertigten Gels in Cyclomethicone und gewünschtenfalls eines Gelaktivators, wie z. B. Propylencarbonat, Ethanol oder Wasser, bereit. Weitere geeignete Verdickungsmittel sind pyrogene Kieselsäuren, z. B. die Handelsprodukte der Aerosil^{®}-Serie von Degussa.

Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien als Pulver oder in Form eines Gels in einer Ölkomponente, bevorzugt in Cyclomethicone und/oder einer Nichtsilicon-Ölkomponente, wie z. B. Propylencarbonat, bereit. Die Gelbildung erfolgt durch den Zusatz geringer Mengen an Aktivatoren, wie insbesondere Ethanol oder Propylencarbonat, aber auch Wasser. Derartige Gele sind beispielsweise unter der Handelsbezeichnung Bentone^{®} oder Thixogel erhältlich. Bevorzugte erfindungsgemäße Zusammensetzungen enthalten mindestens einen Aktivator in einer Gesamtmenge von 0,1 - 7 Gew.-%, bevorzugt 0,3 - 5 Gew.-%, außerordentlich bevorzugt 1,6 - 3 Gew.-%, jeweils bezogen auf das Gesamtgewicht der treibmittelfreien erfindungsgemäßen Zusammensetzung. Weitere bevorzugte erfindungsgemäße Zusammensetzungen enthalten mindestens einen Aktivator, ausgewählt aus Ethanol, Propylencarbonat und Wasser sowie Mischungen hiervon, in einer Gesamtmenge von 0,1 - 3 Gew.-%, bevorzugt 0,3 - 1,6 Gew.-%, jeweils bezogen auf das Gesamtgewicht der treibmittelfreien erfindungsgemäßen Zusammensetzung. Erfindungsgemäß bevorzugte Zusammensetzungen sind dadurch gekennzeichnet, dass sie mindestens ein hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der treibmittelfreien erfindungsgemäßen Zusammensetzung, enthalten.

Weitere erfindungsgemäß bevorzugte lipophile Verdickungsmittel sind ausgewählt aus pyrogenen Kieselsäuren, z. B. den Handelsprodukten der Aerosil^{®}-Serie von Evonik Degussa. Besonders bevorzugt sind hydrophobierte pyrogene Kieselsäuren, besonders bevorzugt Silica Silylate und Silica Dimethyl Silylate.

Erfindungsgemäß bevorzugte Zusammensetzungen sind dadurch gekennzeichnet, dass sie mindestens eine pyrogene Kieselsäure, bevorzugt mindestens eine hydrophobierte pyrogene Kieselsäure, in einer Gesamtmenge von 0,5 - 10 Gew.-%, bevorzugt 0,8 - 5 Gew.-%, besonders bevor-. zugt 1 - 4 Gew.-%, außerordentlich bevorzugt 2 - 3 Gew.-%, jeweils bezogen auf das Gesamtgewicht der treibmittelfreien erfindungsgemäßen Zusammensetzung, enthalten. Erfindungsgemäß bevorzugte Zusammensetzungen sind dadurch gekennzeichnet, dass sie mindestens eine hydrophile pyrogene Kieselsäure, bevorzugt in einer Gesamtmenge von 0,1 - 10 Gew.-%, besonders bevorzugt 0,3 - 5 Gew.-%, bevorzugt 1 - 2 Gew.-%, außerordentlich bevorzugt 0,7 1 Gew.-%, jeweils bezogen auf das Gesamtgewicht der treibmittelfreien erfindungsgemäßen Zusammensetzung, enthalten. Bevorzugte hydrophile pyrogene Kieselsäuren sind unter den Handelsbezeichnungen Aerosil 200 und Aerosil 300 erhältlich.

Weitere erfindungsgemäß bevorzugte Zusammensetzungen sind dadurch gekennzeichnet, dass sie mindestens eine hydrophobierte pyrogene Kieselsäure und mindestens eine hydrophile Kiesalsäure (INCI: Silica) enthalten.

Weitere erfindungsgemäß bevorzugte lipophile Verdickungsmittel sind ausgewählt aus Ethylene/ Propylene/Styrene Copolymeren und Butylene/Ethylene/Styrene Copolymeren. Besonders bevorzugt werden die Copolymere als vorverdicktes Öl-basiertes Gel eingesetzt.

Erfindungsgemäß bevorzugte Zusammensetzungen sind dadurch gekennzeichnet, dass sie mindestens ein Ethylene/Propylene/Styrene Copolymer und/oder Butylene/Ethylene/Styrene Copolymer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der treibmittelfreien erfindungsgemäßen Zusammensetzung, enthalten. Weitere bevorzugte erfindungsgemäße Zusammensetzungen sind dadurch gekennzeichnet, dass sie mindestens ein Silicongummi enthalten. Überraschend wurde festgestellt, dass durch den Zusatz eines Silicongummis die schweißhemmende und deodorierende Wirkung der erfindungsgemäßen Zusammensetzungen weiter verlängert werden kann. Ohne an diese Theorie gebunden sein zu wollen, wird vermutet, dass das Silicongummi auf der Haut einen Film bildet, durch den die schweißhemmenden Wirkstoffpartikel besser auf der Haut haften.

Ein erfindungsgemäß besonders bevorzugtes Silicongummi ist ausgewählt aus Siliconpolymeren mit der INCI-Bezeichnung Dimethiconol. Bevorzugt werden diese Dimethiconole in einer niedrigkonzentrierten Lösung in Cyclomethicone oder Dimethicone mit einer kinematischen Viskosität von 0,65 cSt bis maximal 10 cSt eingesetzt. Besonders bevorzugte Dimethiconole sind von Dow Corning unter den Handelsnamen Dow Corning 1401, Dow Corning 1403 und Dow Corning 1501 erhältlich; diese Produkte enthalten 10 bis 13 Gew.-% Dimethiconol in Cyclomethicone oder Dimethicone.

Erfindungsgemäß bevorzugte Zusammensetzungen sind dadurch gekennzeichnet, dass sie mindestens ein Silicongummi in einer Gesamtmenge von 0,01 - 1,0 Gew.-%, bevorzugt 0,05 - 0,2 Gew.-%, besonders bevorzugt 0,1 - 0,15 Gew.-%, jeweils bezogen auf das Gesamtgewicht der treibmittelfreien erfindungsgemäßen Zusammensetzung, enthalten.

Die erfindungsgemäßen Zusammensetzungen enthalten mindestens einen Antitranspirant-Wirkstoff.

Bevorzugte Antitranspirant-Wirkstoffe sind ausgewählt aus den wasserlöslichen adstringierenden anorganischen und organischen Salzen des Aluminiums und Zinks bzw. beliebigen Mischungen dieser Salze.

Alumosilicate und Zeolithe zählen erfindungsgemäß nicht zu den Antitranspirant-Wirkstoffen. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorohydrex-Propylenglycol (PG) oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG oder Aluminiumsesquichlorohydrex-PEG, Aluminium-PG-dichlorohydrex oder Aluminium-PEGdichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat und Zinksulfat.

Erfindungsgemäß besonders bevorzugte Antitranspirant-Wirkstoffe sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminiumsalze müssen nicht notwendigerweise als sprühgetrocknetes Pulver eingesetzt werden. Erfindungsgemäß ebenfalls bevorzugte schweißhemmende Wirkstoffe sind nichtwässrige Lösungen oder Solubilisate eines aktivierten schweißhemmenden Aluminiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminiumsalze mit einem mehrwertigen Alkohol, die 20 - 50 Gew.-%, besonders bevorzugt 20 - 42 Gew.-%, aktiviertes schweißhemmendes Aluminiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind aktivierte Aluminiumsalze, wie sie beispielsweise in US 6245325 oder US 6042816 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Aluminium - Gewichtsverhältnis von 2:1 - 1:20 und bevorzugt 1:1 bis 1:10 bereitzustellen, sowie ein wasserlösliches Calciumsalz in einer solchen Menge, um ein Ca:Al-Gewichtsverhältnis von 1:1 - 1:28 und bevorzugt 1:2 - 1:25 bereitzustellen.

Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt. Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Calciumsalz, dass das Ca:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminiumsalze, wie sie beispielsweise in US 6902723 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Weitere bevorzugte schweißhemmende Wirkstoffe sind in US 6663854 und US 20040009133 offenbart.

Die schweißhemmenden Wirkstoffe können sowohl in solubilisierter als auch in ungelöster, suspendierter Form vorliegen.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Wirkstoffpartikel weisen eine volumenmittlere Partikelgröße von 0,2 - 220 µm, bevorzugt 3 - 60 µm, besonders bevorzugt 4 - 25 µm, weiterhin bevorzugt 5 - 20 µm und außerordentlich bevorzugt 10 - 15,5 µm, auf. Bevorzugte Aluminiumsalze weisen ein molares Metall-zu-Chlorid-Verhältnis von 1,9 -2,1, bzw. für Sesquichlorohydrate von 1,5:1-1,8:1 auf.

Die Antitranspirant-Wirkstoffe können als nicht-wässrige Lösungen oder als glycolische Solubilisate eingesetzt werden.

Besonders bevorzugte erfindungsgemäße Zusammensetzungen sind dadurch gekennzeichnet, dass der mindestens eine Antitranspirant-Wirkstoff in einer Menge von 5 - 40 Gew.-%, bevorzugt 10 - 35 Gew.-%, besonders bevorzugt 15 - 28 Gew.-% und außerordentlich bevorzugt 23-27 Gew.-%, enthalten ist, bezogen auf das Gesamtgewicht der kristallwasserfreien Aktivsubstanz (USP) in der treibmittelfreien Gesamtzusammensetzung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit/Reheis, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter der Bezeichnung Reach^{®} 301 wird ein Aluminiumsesquichlorohydrat von Reheis angeboten, das ebenfalls besonders bevorzugt ist.

Die erfindungsgemäßen Zusammensetzungen enthalten mindestens ein Öl als Trägerfluid.

Erfindungsgemäß bevorzugte Antitranspirant-Zusammensetzungen enthalten 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte treibmittelfreie Zusammensetzung, an mindestens einem unter Normalbedingungen flüssigen kosmetischen Öl. Auch eine Gesamtmenge an unter Normalbedingungen flüssigen kosmetischen Ölen von 53, 55, 58, 60, 63, 65, 68, 70, 73, 75, 78 oder 80 Gew.-%, jeweils bezogen auf die gesamte treibmittelfreie Zusammensetzung, kann erfindungsgemäß besonders bevorzugt sein, wobei eine Gesamtmenge von 53 - 73 Gew.-% besonders bevorzugt ist.

Bei den kosmetischen Ölen unterscheidet man flüchtige und nicht-flüchtige Öle. Unter nichtflüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 12 - 12000 Pa (0,1 - 90 mm Hg), besonders bevorzugt 13 - 8000 Pa, außerordentlich bevorzugt 30 - 3000 Pa, weiterhin bevorzugt 100 - 400 Pa, aufweisen.

Erfindungsgemäß bevorzugte kosmetische Öle sind ausgewählt aus flüchtigen Siliconölen, zu denen z. B. Dialkyl- und Alkylarylsiloxane, wie beispielsweise Cyclotetrasiloxan, Cyclopentasiloxan, Cyclohexasiloxan, Dimethylpolysiloxan, niedermolekulares Phenyl Trimethicone und Methylphenylpolysiloxan, aber auch Hexamethyldisiloxan, Octamethyltrisiloxan und Decamethyltetrasiloxan zählen. Besonders bevorzugt sind flüchtige Siliconöle, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning (Dampfdruck bei 20°C ca. 13 - 15 Pa) enthalten sind. Ebenfalls besonders bevorzugt sind flüchtige lineare Siliconöle mit 2 - 10 Siloxaneinheiten, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Ein weiteres bevorzugtes flüchtiges Siliconöl ist ein niedermolekulares Phenyl Trimethicone mit einem Dampfdruck bei 20°C von etwa 2000 Pa, wie es z. B. von GE Bayer Silicones/Momentive unter der Bezeichnung Baysilone Fluid PD 5 erhältlich ist. Flüchtige Siliconöle sind hervorragend geeignete Trägeröle für erfindungsgemäß bevorzugte Antitranspirant-Zusammensetzungen, da sie ihnen ein angenehmes Hautgefühl und eine geringe Kleideranschmutzung verleihen. Erfindungsgemäß besonders bevorzugte Antitranspirant-Zusammensetzungen sind daher durch einen Gehalt an mindestens einem flüchtigen Siliconöl von 10 - 95 Gew.-%, bevorzugt 30 - 80 Gew.-%, besonders bevorzugt 40 - 70 Gew.-%, außerordentlich bevorzugt 50 - 60 Gew.-%, jeweils bezogen auf die gesamte treibmittelfreie Zusammensetzung, gekennzeichnet.

Neben oder an Stelle des mindestens einen flüchtigen Siliconöls kann auch mindestens ein flüchtiges Nichtsiliconöl enthalten sein. Bevorzugte flüchtige Nichtsiliconöle sind ausgewählt aus C₈-C₁₆-Isoparaffinen, insbesondere aus Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von etwa 10 - 400 Pa, bevorzugt 13 - 300 Pa. Dieses mindestens eine flüchtige Nichtsiliconöl ist bevorzugt in einer Gesamtmenge von 10 - 95 Gew.-%, bevorzugt 20 - 70 Gew.-%, besonders bevorzugt 25 - 50 Gew.-%, außerordentlich bevorzugt 30 - 40 Gew.-%, jeweils bezogen auf die gesamte treibmittelfreie Zusammensetzung, enthalten.

Aufgrund des trockeneren Hautgefühls und der schnelleren Wirkstofffreisetzung sind als Trägeröl flüchtige Siliconöle, Isoparaffine, insbesondere Isodecan, Isoundecan, Isododecan, Isotridecan, Isotetradecan, Isopentadecan, Isohexadecan und Isoeicosan, sowie Mischungen von flüchtigen Siliconölen und Isoparaffinen, insbesondere Isododecan, Isohexadecan oder Isoeicosan, besonders bevorzugt.

Erfindungsgemäß bevorzugte Zusammensetzungen sind dadurch gekennzeichnet, dass das mindestens eine unter Normalbedingungen flüssige Träger-Öl b) mindestens ein Isoparaffinöl, insbesondere Isodecan, Isoundecan, Isododecan, Isotridecan, Isotetradecan, Isopentadecan, Isohexadecan und Isoeicosan, umfasst.

Weitere erfindungsgemäß bevorzugte Zusammensetzungen sind dadurch gekennzeichnet, dass das unter Normalbedingungen flüssige Träger-Öl b) eine Mischung aus b)i) einem flüchtigen Siliconöl, ausgewählt aus Cyclomethicone und linearen Polydimethylsiloxanen mit 2 - 10 Siloxaneinheiten, und b)ii) mindestens einem Isoparaffinöl, ausgewählt aus Isodecan, Isoundecan, Isododecan, Isotridecan, Isotetradecan, Isopentadecan, Isohexadecan und Isoeicosan, umfasst. Neben den vorgenannten, üblicherweise als "flüchtigen" Siliconölen bezeichneten Substanzen sowie neben den vorgenannten flüchtigen Nichtsiliconölen können erfindungsgemäß besonders bevorzugte Antitranspirant-Zusammensetzungen weiterhin mindestens ein nichtflüchtiges kosmetisches Öl, ausgewählt aus nichtflüchtigen Siliconölen und nichtflüchtigen Nichtsiliconölen, enthalten. Das mindestens eine nichtflüchtige Öl gleicht den negativen Effekt des flüchtigen Öls auf das Rückstandsverhalten erfindungsgemäß bevorzugter Antitranspirant-Zusammensetzungen aus. Durch die relativ schnelle Verdunstung der flüchtigen Öle können feste, unlösliche Bestandteile, insbesondere die Antitranspirantwirkstoffe, auf der Haut als unschöner Rückstand sichtbar werden. Diese Rückstände können erfolgreich mit einem nichtflüchtigen Öl maskiert werden. Außerdem können mit einem Gemisch aus nichtflüchtigem und flüchtigem Öl Parameter wie Hautgefühl, Sichtbarkeit des Rückstands und Stabilität der Suspension feinreguliert und besser an die Bedürfnisse der Verbraucher angepasst werden.

Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 6 - 50 cSt oder auch 5 - 10 cSt, und Baysilon^{®} 350 M (mit einer kinematischen Viskosität (25°C) von etwa 350 cSt).

Erfindungsgemäß ebenfalls bevorzugte Siliconöle sind ausgewählt aus Siliconen der Formel (Sil-1), wobei x ausgewählt ist aus ganzen Zahlen von 1 - 20.

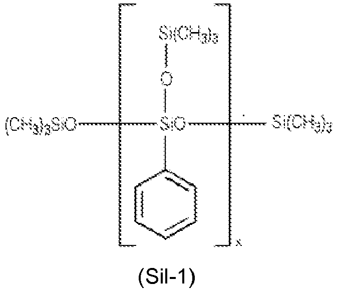

Ein bevorzugtes Siliconöl der Formel (Sil-1) ist unter der INCI-Bezeichnung Phenyl Trimethicone in verschiedenen Qualitäten, Viskositäten und Flüchtigkeiten erhältlich. Ein nicht-flüchtiges Phenyl Trimethicone ist beispielsweise von Dow Corning unter der Bezeichnung Dow Corning 556 erhältlich.

Erfindungsgemäß besonders bevorzugte nichtflüchtige Nichtsiliconöle sind ausgewählt aus den Estern der linearen oder verzweigten gesättigten oder nichtflüchtige Nichtsiliconöle ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Hierunter sind Isopropylpalmitat, Isopropylstearat, Isopropylmyristat, 2-Ethylhexylpalmitat (Cegesoft^{®} C 24) und 2-Ethylhexylstearat (Cetiol^{®} 868) außerordentlich bevorzugt. Ebenfalls bevorzugt sind Hexyldecylstearat (Eutanol^{®} G16S), Hexyldecyllaurat, Isononylisononanoat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat und Erucylerucat.

Weitere erfindungsgemäß bevorzugte nichtflüchtige Nichtsiliconöle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD, wobei Benzoesäure-C12-C15-alkylester außerordentlich bevorzugt sind. Ein weiteres erfindungsgemäß bevorzugtes nichtflüchtiges Nichtsiliconöl ist Triethylcitrat. Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von Cognis), gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16), Octyldodecanol (Eutanol^{®} G) und 2-Ethylhexylalkohol.

Weitere bevorzugte nichtflüchtige Nichtsiliconöle sind ausgewählt aus Mischungen aus Guerbetalkoholen und Guerbetalkoholestern, z.B. dem Handelsprodukt Cetiol^{®} PGL (Hexyldecanol und Hexyldecyllaurat).

Weitere erfindungsgemäß bevorzugte nichtflüchtige Nichtsiliconöle sind ausgewählt aus den Einfach- und Mehrfachestern von Milchsäure, Citronensäure, Weinsäure oder Adipinsäure mit einem zwei-, drei- oder vierwertigen Alkohol mit 2 bis 9 Kohlenstoffatomen. Besonders bevorzugte Ester dieser Art sind ausgewählt aus Ethylenglycolmonolactat, Ethylenglycolmonocitrat, Ethylenglycolmonotartrat, Ethylenglycolmonoadipat, Ethylenglycoldilactat, Ethylenglycoldicitrat, Ethylenglycolditartrat, Ethylenglycoldiadipat, 1,2-Propylenglycolmonolactat, 1,2-Propylenglycolmonocitrat, 1,2-Propylenglycolmonotartrat, 1,2-Propylenglycolmonoadipat, 1,2-Propylenglycoldilactat, 1,2-Propylenglycoldicitrat, 1,2-Propylenglycolditartrat, 1,2-Propylenglycoldiadipat, 1,3-Propylenglycolmonolactat, 1,3-Propylenglycolmonocitrat, 1,3-Propylenglycolmonotartrat, 1,3-Propylenglycolmonoadipat, 1,3-Propylenglycoldilactat, 1,3-Propylenglycoldicitrat, 1,3-Propylenglycolditartrat, 1,3-Propylenglycoldiadipat, 1,2-Butylenglycolmonolactat, 1,2-Butylenglycolmonocitrat, 1,2-Butylenglycolmonotartrat, 1,2-Butylenglycolmonoadipat, 1,2-Butylenglycoldilactat, 1,2-Butylenglycoldicitrat, 1,2-Butylenglycolditartrat, 1,2-Butylenglycoldiadipat, 1,3-Butylenglycolmonolactat, 1,3-Butylenglycolmonocitrat, 1,3-Butylenglycolmonotartrat, 1,3-Butylenglycolmonoadipat, 1,3-Butylenglycoldilactat, 1,3-Butylenglycoldicitrat, 1,3-Butylenglycolditartrat, 1,3-Butylenglycoldiadipat, 1,4-Butylenglycolmonolactat, 1,4-Butylenglycolmonocitrat, 1,4-Butylenglycolmonotartrat, 1,4-Butylenglycolmonoadipat, 1,4-Butylenglycoldilactat, 1,4-Butylenglycoldicitrat, 1,4-Butylenglycolditartrat, 1,4-Butylenglycoldiadipat, 1,2-Pentandiolmonolactat, 1,2-Pentandiolmonocitrat, 1,2-Pentandiolmonotartrat, 1,2-Pentandiolmonoadipat, 1,2-Pentandioldilactat, 1,2-Pentandioldicitrat, 1,2-Pentandiolditartrat, 1,2-Pentandioldiadipat, 1,3-Pentandiolmonolactat, 1,3-Pentandiolmonocitrat, 1,3-Pentandiolmonotartrat, 1,3-Pentandiolmonoadipat, 1,3-Pentandioldilactat, 1,3-Pentandioldicitrat, 1,3-Pentandiolditartrat, 1,3-Pentandioldiadipat, 1,4-Pentandiolmonolactat, 1,4-Pentandiolmonocitrat, 1,4-Pentandiolmonotartrat, 1,4-Pentandiolmonoadipat, 1,4-Pentandioldilactat, 1,4-Pentandioldicitrat, 1,4-Pentandiolditartrat, 1,4-Pentandioldiadipat, 1,5-Pentandiolmonolactat, 1,5-Pentandiolmonocitrat, 1,5-Pentandiolmonotartrat, 1,5-Pentandiolmonoadipat, 1,5-Pentandioldilactat, 1,5-Pentandioldicitrat, 1,5-Pentandiolditartrat, 1,5-Pentandioldiadipat, 1,2-Hexandiolmonolactat, 1,2-Hexandiolmonocitrat, 1,2-Hexandiolmonotartrat, 1,2-Hexandiolmonoadipat, 1,2-Hexandioldilactat, 1,2-Hexandioldicitrat, 1,2-Hexandiolditartrat, 1,2-Hexandioldiadipat, 1,3-Hexandiolmonolactat, 1,3-Hexandiolmonocitrat, 1,3-Hexandiolmonotartrat, 1,3-Hexandiolmonoadipat, 1,3-Hexandioldilactat, 1,3-Hexandioldicitrat, 1,3-Hexandiolditartrat, 1,3-Hexandioldiadipat, 1,4-Hexandiolmonolactat, 1,4-Hexandiolmonocitrat, 1,4-Hexandiolmonotartrat, 1,4-Hexandiolmonoadipat, 1,4-Hexandioldilactat, 1,4-Hexandioldicitrat, 1,4-Hexandiolditartrat, 1,4-Hexandioldiadipat, 1,5-Hexandiolmonolactat, 1,5-Hexandiolmonocitrat, 1,5-Hexandiolmonotartrat, 1,5-Hexandiolmonoadipat, 1,5-Hexandioldilactat, 1,5-Hexandioldicitrat, 1,5-Hexandiolditartrat, 1,5-Hexandioldiadipat, 1,6-Hexandiolmonolactat, 1,6-Hexandiolmonocitrat, 1,6-Hexandiolmonotartrat, 1,6-Hexandiolmonoadipat, 1,6-Hexandioldilactat, 1,6-Hexandioldicitrat, 1,6-Hexandiolditartrat, 1,6-Hexandioldiadipat, 2-Ethylhexan-1,2-diolmonolactat, 2-Ethylhexan-1,2-diolmonocitrat, 2-Ethylhexan-1,2-diolmonotartrat, 2-Ethylhexan-1,2-diolmonoadipat, 2-Ethylhexan-1,2-dioldilactat, 2-Ethylhexan-1,2-dioldicitrat, 2-Ethylhexan-1,2-diolditartrat, 2-Ethylhexan-1,2-dioldiadipat, 2-Ethylhexan-1,3-diolmonolactat, 2-Ethylhexan-1,3-diolmonocitrat, 2-Ethylhexan-1,3-diolmonotartrat, 2-Ethylhexan-1,3-diolmonoadipat, 2-Ethylhexan-1,3-dioldilactat, 2-Ethylhexan-1,3-dioldicitrat, 2-Ethylhexan-1,3-diolditartrat, 2-Ethylhexan-1,3-dioldiadipat, 2-Ethylhexan-1,4-diolmonolactat, 2-Ethylhexan-1,4-diolmonocitrat, 2-Ethylhexan-1,4-diolmonotartrat, 2-Ethylhexan-1,4-diolmonoadipat, 2-Ethylhexan-1,4-dioldilactat, 2-Ethylhexan-1,4-dioldicitrat, 2-Ethylhexan-1,4-diolditartrat, 2-Ethylhexan-1,4-dioldiadipat, 2-Ethylhexan-1,5-diolmonolactat, 2-Ethylhexan-1,5-diolmonocitrat, 2-Ethylhexan-1,5-diolmonotartrat, 2-Ethylhexan-1,5-diolmonoadipat, 2-Ethylhexan-1,5-dioldilactat, 2-Ethylhexan-1,5-dioldicitrat, 2-Ethylhexan-1,5-diolditartrat, 2-Ethylhexan-1,5-dioldiadipat, 2-Ethylhexan-1,6-diolmonolactat, 2-Ethylhexan-1,6-diolmonocitrat, 2-Ethylhexan-1,6-diolmonotartrat, 2-Ethylhexan-1,6-diolmonoadipat, 2-Ethylhexan-1,6-dioldilactat, 2-Ethylhexan-1,6-dioldicitrat, 2-Ethylhexan-1,6-diolditartrat, 2-Ethylhexan-1,6-dioldiadipat, 1,2-Heptandiolmonolactat, 1,2-Heptandiolmonocitrat, 1,2-Heptandiolmonotartrat, 1,2-Heptandiolmonoadipat, 1,2-Heptandioldilactat, 1,2-Heptandioldicitrat, 1,2-Heptandiolditartrat, 1,2-Heptandioldiadipat, 1,3-Heptandiolmonolactat, 1,3-Heptandiolmonocitrat, 1,3-Heptandiolmonotartrat, 1,3-Heptandiolmonoadipat, 1,3-Heptandioldilactat, 1,3-Heptandioldicitrat, 1,3-Heptandiolditartrat, 1,3-Heptandioldiadipat, 1,4-Heptandiolmonolactat, 1,4-Heptandiolmonocitrat, 1,4-Heptandiolmonotartrat, 1,4-Heptandiolmonoadipat, 1,4-Heptandioldilactat, 1,4-Heptandioldicitrat, 1,4-Heptandiolditartrat, 1,4-Heptandioldiadipat, 1,5-Heptandiolmonolactat, 1,5-Heptandiolmonocitrat, 1,5-Heptandiolmonotartrat, 1,5-Heptandiolmonoadipat, 1,5-Heptandioldilactat, 1,5-Heptandioldicitrat, 1,5-Heptandiolditartrat, 1,5-Heptandioldiadipat, 1,6-Heptandiolmonolactat, 1,6-Heptandiolmonocitrat, 1,6-Heptandiolmonotartrat, 1,6-Heptandiolmonoadipat, 1,6-Heptandioldilactat, 1,6-Heptandioldicitrat, 1,6-Heptandiolditartrat, 1,6-Heptandioldiadipat, 1,7-Heptandiolmonolactat, 1,7-Heptandiolmonocitrat, 1,7-Heptandiolmonotartrat, 1,7-Heptandiolmonoadipat, 1,7-Heptandioldilactat, 1,7-Heptandioldicitrat, 1,7-Heptandiolditartrat, 1,7-Heptandioldiadipat, 1,2-Octandiolmonolactat, 1,2-Octandiolmonocitrat, 1,2-Octandiolmonotartrat, 1,2-Octandiolmonoadipat, 1,2-Octandioldilactat, 1,2-Octandioldicitrat, 1,2-Octandiolditartrat, 1,2-Octandioldiadipat, 1,3-Octandiolmonolactat, 1,3-Octandiolmonocitrat, 1,3-Octandiolmonotartrat, 1,3-Octandiolmonoadipat, 1,3-Octandioldilactat, 1,3-Octandioldicitrat, 1,3-Octandiolditartrat, 1,3-Octandioldiadipat, 1,4-Octandiolmonolactat, 1,4-Octandiolmonocitrat, 1,4-Octandiolmonotartrat, 1,4-Octandiolmonoadipat, 1,4-Octandioldilactat, 1,4-Octandioldicitrat, 1,4-Octandiolditartrat, 1,4-Octandioldiadipat, 1,5-Octandiolmonolactat, 1,5-Octandiolmonocitrat, 1,5-Octandiolmonotartrat, 1,5-Octandiolmonoadipat, 1,5-Octandioldilactat, 1,5-Octandioldicitrat, 1,5-Octandiolditartrat, 1,5-Octandioldiadipat, 1,6-Octandiolmonolactat, 1,6-Octandiolmonocitrat, 1,6-Octandiolmonotartrat, 1,6-Octandiolmonoadipat, 1,6-Octandioldilactat, 1,6-Octandioldicitrat, 1,6-Octandiolditartrat, 1,6-Octandioldiadipat, 1,7-Octandiolmonolactat, 1,7-Octandiolmonocitrat, 1,7-Octandiolmonotartrat, 1,7-Octandiolmonoadipat, 1,7-Octandioldilactat, 1,7-Octandioldicitrat, 1,7-Octandiolditartrat, 1,7-Octandioldiadipat, 1,8-Octandiolmonolactat, 1,8-Octandiolmonocitrat, 1,8-Octandiolmonotartrat, 1,8-Octandiolmonoadipat, 1,8-Octandioldilactat, 1,8-Octandioldicitrat, 1,8-Octandiolditartrat, 1,8-Octandioldiadipat, 2-Methyl-1,3-propandiolmonolactat, 2-Methyl-1,3-propandiolmonocitrat, 2-Methyl-1,3-propandiolmonotartrat, 2-Methyl-1,3-propandiolmonoadipat, 2-Methyl-1,3-propandioldilactat, 2-Methyl-1,3-propandioldicitrat, 2-Methyl-1,3-propandiolditartrat, 2-Methyl-1,3-propandioldiadipat, Dipropylenglycolmonolactat, Dipropylenglycolmonotartrat, Dipropylenglycolmonocitrat, Dipropylenglycolmonoadipat, Dipropylenglycoldilactat, Dipropylenglycolditartrat, Dipropylenglycoldicitrat, Dipropylenglycoldiadipat, Glycerinmonolactat, Glycerinmonotartrat, Glycerinmonocitrat, Glycerinmonoadipat, Glycerindilactat, Glycerinditartrat, Glycerindicitrat, Glycerindiadipat, Glycerintrilactat, Glycerintritartrat, Glycerintricitrat, Glycerintriadipat, Diglycerinmonolactat, Diglycerinmonotartrat, Diglycerinmonocitrat, Diglycerinmonoadipat, Diglycerindilactat, Diglycerinditartrat, Diglycerindicitrat, Diglycerindiadipat, Diglycerintrilactat, Diglycerintritartrat, Diglycerintricitrat, Diglycerintriadipat, Tripropylenglycolmonolactat, Tripropylenglycolmonotartrat, Tripropylenglycolmonocitrat, Tripropylenglycolmonoadipat, Tripropylenglycoldilactat, Tripropylenglycolditartrat, Tripropylenglycoldicitrat, Tripropylenglycoldiadipat, Tripropylenglycoltrilactat, Tripropylenglycoltritartrat, Tripropylenglycoltricitrat, Tripropylenglycoltriadipat, Triglycerinmonolactat, Triglycerinmonotartrat, Triglycerinmonocitrat, Triglycerinmonoadipat, Triglycerindilactat, Triglycerinditartrat, Triglycerindicitrat, Triglycerindiadipat, Triglycerintrilactat, Triglycerintritartrat, Triglycerintricitrat, Triglycerintriadipat, 1,2,6-Hexantriolmonolactat, 1,2,6-Hexantriolmonotartrat, 1,2,6-Hexantriolmonocitrat, 1,2,6-Hexantriolmonoadipat, 1,2,6-Hexantrioldilactat, 1,2,6-Hexantriolditartrat, 1,2,6-Hexantrioldicitrat, 1,2,6-Hexantrioldiadipat, 1,2,6-Hexantrioltrilactat, 1,2,6-Hexantrioltritartrat, 1,2,6-Hexantrioltricitrat, 1,2,6-Hexantrioltriadipat, Trimethylolpropanmonolactat, Trimethylolpropanmonotartrat, Trimethylolpropanmonocitrat, Trimethylolpropanmonoadipat, Trimethylolpropandilactat, Trimethylolpropanditartrat, Trimethylolpropandicitrat, Trimethylolpropandiadipat, Trimethylolpropantrilactat, Trimethylolpropantritartrat, Trimethylolpropantricitrat, Trimethylolpropantriadipat, Trimethylolethanmonolactat, Trimethylolethanmonotartrat, Trimethylolethanmonocitrat, Trimethylolethanmonoadipat, Trimethylolethandilactat, Trimethylolethanditartrat, Trimethylolethandicitrat, Trimethylolethandiadipat, Trimethylolethantrilactat, Trimethylolethantritartrat, Trimethylolethantricitrat und Trimethylolethantriadipat, sowie Mischungen hiervon.

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin mit verzweigten Fettsäureresten.

Weitere erfindungsgemäß besonders bevorzugte nichtflüchtige Nichtsiliconöle sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC), Di-n-octylcarbonat, Di-n-dodecylcarbonat, Di-(2-ethylhexyl)carbonat oder die Ester gemäß der Lehre der DE 19756454 A. Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₆₋Alkanolen oder mit mehrwertigen linearen oder verzweigten C₂-C₆-Alkanolen. Es kann erfindungsgemäß bevorzugt sein, Mischungen der vorgenannten Öle einzusetzen. Erfindungsgemäß bevorzugte Zusammensetzungen sind dadurch gekennzeichnet, dass das mindestens eine unter Normalbedingungen flüssige Träger-Öl b) ausgewählt ist aus flüchtigen cyclischen oder linearen Siliconölen, nichtflüchtigen höhermolekularen linearen Dimethylpolysiloxanen, Estern von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Benzoesäureestern von linearen oder verzweigten C₆₋₂₂-Alkanolen, den C₆-C₂₂ Fetalkoholestern einwertiger oder mehrwertiger C₂-C₇-Hydroxycarbonsäuren, den Anlagerungsprodukten von Ethylenoxid und/oder Propylenoxid an ein- oder mehrwertige C₃₋₂₀-Alkanole, flüssigen Paraffinölen, Isoparaffinölen, insbesondere Isodecan, Isoundecan, Isododecan, Isotridecan, Isotetradecan, Isopentadecan, Isohexadecan und Isoeicosan, synthetischen Kohlenwasserstoffen, wie Polyisobuten oder Polydecene, und alicyclischen Kohlenwasserstoffen, verzweigten gesättigten oder ungesättigten Fettalkoholen mit 6 - 30 Kohlenstoffatomen, Mischungen aus Guerbetalkoholen und Guerbetalkoholestern, den symmetrischen, unsymmetrischen oder cyclischen Estern der Kohlensäure mit Fettalkoholen, Triglyceriden von linearen oder verzweigten, gesättigten oder ungesättigten, gegebenenfalls hydroxylierten C₆₋₃₀-Fettsäuren, Dicarbonsäureestern von linearen oder verzweigten C₂-C₁₀-Alkanolen, Di-n-alkyl-ethern mit insgesamt 12 bis 36, insbesondere 12 bis 24 C-Atomen, sowie Mischungen der vorgenannten Öle.

Weitere erfindungsgemaß bevorzugte Zusammensetzungen sind dadurch gekennzeichnet, dass mindestens ein nichtflüchtiges Nichtsiliconöl in einer Gesamtmenge von 10 - 5 Gew.-%, bevorzugt 15 - 75 Gew.-%, besonders bevorzugt 18 - 50 Gew.-%, außerordentlich bevorzugt 20 - 35 Gew,-%, jeweils bezogen auf die gesamte treibmittelfreie Zusammensetzung, enthalten ist.

### Cyclomethicone-reduzierte Träger

Gemäß einer weiteren, ebenfalls bevorzugten Ausführungsform enthalten die erfindungsgemäßen wasserfreien Antitranspirant-Zusammensetzungen einen geringen Anteil von maximal 2 Gew.-%, bevorzugt maximal 1 Gew.-%, an Cyclomethicone oder sind sogar frei von Cyclomethicone. Als Ersatz für Cyclomethicone sind besonders bevorzugt die C₆-C₁₅-Isoparaffine, insbesondere ausgewählt aus Isononan, Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von etwa B - 400 Pa, bevorzugt 13-300 Pa.

### Cyclomethicone-reduzierte Isoparaffin-Mischungen

Neben dem mindestens einen vorgenannten C₈-C₁₆-Isoparaffin enthalten weitere erfindungsgemäß bevorzugte Antitranspirant-Zusammensetzungen neben 0 bis maximal 2 Gew.-%, bevorzugt maximal 1 Gew.-%, an Cyclomethicone mindestens ein nichtflüchtiges kosmetisches Öl, ausgewählt aus nichtflüchtigen Siliconölen und nichtflüchtigen Nichtsiliconölen. Das mindestens eine nichtflüchtige Öl gleicht den negativen Effekt des flüchtigen Isoparaffins auf das Rückstandsverhalten erfindungsgemäß bevorzugter Antitranspirant-Zusammensetzungen aus. Durch die relativ schnelle Verdunstung der flüchtigen Öle können feste, unlösliche Bestandteile, insbesondere die Antitranspirantwirkstoffe, auf der Haut als unschöner Rückstand sichtbar werden. Diese Rückstände können erfolgreich mit einem nichtflüchtigen Öl maskiert werden. Außerdem können mit einem Gemisch aus nichtflüchtigem und flüchtigem Öl Parameter wie Hautgefühl, Sichtbarkeit des Rückstands und Stabilität der Suspension feinreguliert und besser an die Bedürfnisse der Verbraucher angepasst werden. Hierzu besonders bevorzugte nichtflüchtige Öle sind insbesondere die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukte Finsolv^{®} TN (C₁₂-C₁₅-Alkylbenzoat), C₁₂-C₁₅-Alkyllactat und Di-C₁₂-C₁₃-Alkylmalat geeignet. Weiterhin kann es besonders bevorzugt sein, erfindungsgemäße wasserfreie Antitranspirant-Zusammensetzungen ohne Cyclomethicone und ohne flüchtige lineare Siliconöle zu formulieren. Auch hierzu sind besonders bevorzugt die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv^{®} TN (C₁₂-C₁₅-Alkylbenzoat), C₁₂-C₁₅-Alkyllactat und Di-C₁₂-C₁₃-Alkylmalat geeignet. Erfindungsgemäß besonders bevorzugte Ölmischungen mit 0 bis maximal 2 Gew.-%, bevorzugt 0 bis maximal 1 Gew.-%, an Cyclomethicone sind 2-Ethylhexylpalmitat/Isodecan/Isoundecan/Isododecan/Isotridecan, Hexyldecyllaurat/Isodecan/Isoundecan/Isododecan/Isotridecan, 2-Ethylhexylstearat/Isodecan/Isoundecan/Isododecan/Isotridecan, Isopropylmyristat/Isodecan/Isoundecan/Isododecan/Isotridecan, Isopropylpalmitat/Isononan/Isodecan/Isoundecan/Isododecan/Isotridecan, 2-Ethylhexyllaurat/Isodecan/Isoundecan/Isododecan/Isotridecan, C₁₂-C₁₅-Alkyllactat/Isodecan/Isoundecan/Isododecan/Isotridecan, C₁₂-C₁₅-Alkylbenzoat/Isodecan/Isoundecan/Isododecan/ Isotridecan und Di-C₁₂-C₁₃-Alkylmalat/Isodecan/Isoundecan/ Isododecan/Isotridecan.

In bevorzugten Ölmischungen mit 0 bis maximal 2 Gew.-%, bevorzugt 0 bis maximal 1 Gew.-%, an Cyclomethicone sind die beiden Öltypen (Ester/C₈-C₁₆-Isoparaffin) in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1.

In anderen bevorzugten Ausführungsformen der Erfindung liegt ein Überschuss an C₈-C₁₆-Isoparaffin gegenüber dem/den Ester(n) vor. In diesen Fällen beträgt das Gewichtsverhältnis Ester/ C₈₋₁₆₋Isoparaffin bevorzugt 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylmyristat/C₈-C₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse Isopropylmyristat/C₈₋₁₆₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylpalmitat/C₈-C₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse Isopropylpalmitat/C₈₋₁₆₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen 2-Ethylhexylpalmitat/C₈-C₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse 2-Ethylhexylpalmitat/C₈₋₁₆₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen C₁₂-C₁₅-Alkylbenzoat/C₈-C₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse C₁₂-C₁₅-Alkylbenzoat/C₈₋₁₆₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Ester/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse Ester/C₁₀₋₁₃₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylmyristat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse Isopropylmyristat/C₁₀₋₁₃₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylpalmitat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse Isopropylpalmitat/C₁₀₋₁₃₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen 2-Ethylhexylpalmitat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse 2-Ethylhexylpalmitat/C₁₀₋₁₃₋Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen C₁₂-C₁₅-Alkylbenzoat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse C₁₂-C₁₅-Alkylbenzoat/C₁₀₋₁₃₋Isoparaffin liegen im Bereich von 0,1 bis 0,8, besonders bevorzugt 0,3 bis 0,6, außerordentlich bevorzugt 0,4 bis 0,5.

Besonders bevorzugte erfindungsgemäße Zusammensetzungen sind dadurch gekennzeichnet, dass das unter Normalbedingungen flüssige Träger-ÖI b) eine Mischung b)i) + b)ii) + b)iii) aus b)i) einem Esteröl, ausgewählt aus 2-Ethylhexylpalmitat, Isopropylmyristat und Isopropylpalmitat, und b)ii) mindestens einem Isoparaffinöl, ausgewählt aus Isodecan, Isoundecan, Isododecan, Isotridecan, Isotetradecan, Isopentadecan, Isohexadecan und Isoeicosan, und b)iii) 0 - maximal 1 Gew.-% Cyclomethicone umfasst.

Weitere besonders bevorzugte erfindungsgemäße Zusammensetzungen sind dadurch gekennzeichnet, dass das unter Normalbedingungen flüssige Träger-ÖI b) aus einer Mischung b)i) + b)ii) + b)iii) aus b)i) einem Esteröl, ausgewählt aus 2-Ethylhexylpalmitat, Isopropylmyristat und Isopropylpalmitat, und b)ii) mindestens einem Isoparaffinöl, ausgewählt aus Isodecan, Isoundecan, Isododecan, Isotridecan, Isotetradecan, Isopentadecan, Isohexadecan und Isoeicosan, und b)iii) 0 - maximal 1 Gew.-% Cyclomethicone besteht.

### Ölmischungen mit Triethylcitrat

Weitere bevorzugte erfindungsgemäße Zusammensetzungen sind dadurch gekennzeichnet, dass das unter Normalbedingungen flüssige Trägeröl ausgewählt ist aus einer Mischung von Triethylcitrat und mindestens einem weiteren unter Normalbedingungen flüssigen kosmetischen Öl als Träger, wobei der Gewichtsanteil von Triethylcitrat an der Gesamtmenge an Ölen, bezogen auf die gesamte treibmittelfreie Zusammensetzung, 13 - 50 Gew.-% beträgt und 0 bis weniger als 1 Gew.-% Cyclomethicone, bezogen auf das Gewicht der treibmittelfreien Zusammensetzung, enthalten ist. Bevorzugt beträgt der Gewichtsanteil von Triethylcitrat am Gesamtölgehalt 12 - 40 Gew.-%, besonders bevorzugt 16 - 35 Gew.-%, außerordentlich bevorzugt 20 - 30 Gew.-%. Bei der Bestimmung des Gesamtölgehalts ist das Treibmittel nicht zu berücksichtigen. Erfindungsgemäß besonders bevorzugte Ölmischungen sind Triethylcitrat/2-Ethylhexylpalmitat/ Isodecan/Isoundecan/Isododecan/Isotridecan, Triethylcitrat/Hexyldecyllaurat/Isodecan/Isoundecan/Isododecan/Isotridecan, Triethylcitrat/2-Ethylhexylstearat/Isodecan/Isoundecan/Isododecan/ Isotridecan, Triethylcitrat/Isopropylmyristat/Isodecan/Isoundecan/Isododecan/Isotridecan, Triethylcitrat/Isopropylpalmitat/Isononan/Isodecan/Isoundecan/Isododecan/Isotridecan, Triethylcitrat/ 2-Ethylhexyllaurat/Isodecan/Isoundecan/Isododecan/Isotridecan, Triethylcitrat/C₁₂-C₁₅-Alkyllactat/ Isodecan/Isoundecan/Isododecan/Isotridecan, Triethylcitrat/C₁₂-C₁₅-Alkylbenzoat/Isodecan/Isoundecan/Isododecan/Isotridecan und Triethylcitrat/Di-C₁₂-C₁₃-lkylmalat/Isodecan/Isoundecan/Isododecan/Isotridecan.

In bevorzugten Ölmischungen sind alle drei Öltypen (Triethylcitrat/Ester/C₈-C₁₆-Isoparaffin) in gleichen Gewichtsanteilen enthalten. Weitere bevorzugte Gewichtsverhältnisse Triethylcitrat/ Ester/C₈₋₁₆₋Isoparaffin sind (1-1,3) : (0,6-1) : (1-3). Weitere bevorzugte Gewichtsverhältnisse Triethylcitrat/Ester/C₈-C₁₆-Isoparaffin sind (1-1,3) : 1 : (1-1,5). Weitere bevorzugte Gewichtsverhältnisse Triethylcitrat/Ester/C₈-C₁₆-Isoparaffin sind (1-1,3) : (0,6 - 0,9) : (2,5 - 3), insbesondere 1:0,8:3.

In bevorzugten Ölmischungen sind alle drei Öltypen (Triethylcitrat/Isopropylmyristat/C₈-C₁₆-Isoparaffin) in gleichen Gewichtsanteilen enthalten. Weitere bevorzugte Gewichtsverhältnisse Triethylcitrat/Isopropylmyristat/C₈₋₁₆₋Isoparaffin sind (1-1,3) : (0,6-1) : (1-3). Weitere bevorzugte Gewichtsverhältnisse Triethylcitrat/Isopropylmyristat/C₈-C₁₆-Isoparaffin sind (1-1,3) : 1 : (1-1,5). Weitere bevorzugte Gewichtsverhältnisse Triethylcitrat/Isopropylmyristat/C₈-C₁₆-Isoparaffin sind (1-1,3):(0,6-0,9):(2,5-3), insbesondere 1:0,8:3.

In bevorzugten Ölmischungen sind alle drei Öltypen (Triethylcitrat/Isopropylpalmitat/C₈-C₁₆-Isoparaffin) in gleichen Gewichtsanteilen enthalten. Weitere bevorzugte Gewichtsverhältnisse Triethylcitrat/Isopropylpalmitat/C₈₋₁₆₋Isoparaffin sind (1-1,3) : (0,6-1) : (1-3). Weitere bevorzugte Gewichtsverhältnisse Triethylcitrat/Isopropylpalmitat/C₈-C₁₆-Isoparaffin sind (1-1,3) : 1 : (1-1,5). Weitere bevorzugte Gewichtsverhältnisse Triethylcitrat/Isopropylpalmitat/C₈-C₁₆-Isoparaffin sind (1-1,3):(0,6-0,9):(2,5-3), insbesondere 1 : 0,8 : 3.

In bevorzugten Ölmischungen sind alle drei Öltypen (Triethylcitrat/C₁₂-C₁₅-Alkylbenzoat/C₈-C₁₆-Isoparaffin) in gleichen Gewichtsanteilen enthalten. Weitere bevorzugte Gewichtsverhältnisse Triethylcitrat/ C₁₂-C₁₅-Alkylbenzoat/C₈₋₁₆₋Isoparaffin sind (1-1,3) : (0,6-1) : (1-3). Weitere bevorzugte Gewichtsverhältnisse Triethylcitrat/C₁₂-C₁₅-Alkylbenzoat/C₈-C₁₆-Isoparaffin sind (1-1,3) : 1 : (1-1,5). Weitere bevorzugte Gewichtsverhältnisse Triethylcitrat/C₁₂-C₁₅-Alkylbenzoat/C₈-C₁₆-Isoparaffin sind (1-1,3):(0,6-0,9):(2,5-3), insbesondere 1:0,8:3.

In weiteren bevorzugten Ölmischungen sind alle drei Öltypen (Triethylcitrat/Ester/C₁₀-C₁₃-Isoparaffin) in gleichen Gewichtsanteilen enthalten. Weitere bevorzugte Gewichtsverhältnisse Triethylcitrat/Ester/C₁₀₋₁₃-Isoparaffin sind (1-1,3) : (0,6-1) : (1-3). Weitere bevorzugte Gewichtsverhältnisse Triethylcitrat/Ester/C₁₀-C₁₃-Isoparaffin sind (1-1,3) : 1 : (1-1,5). Weitere bevorzugte Gewichtsverhältnisse Triethylcitrat/Ester/ C₁₀-C₁₃-Isoparaffin sind (1-1,3) : (0,6 - 0,9) : (2,5 - 3), insbesondere 1:0,8:3.

In bevorzugten Ölmischungen sind alle drei Öltypen (Triethylcitrat/Isopropylmyristat/C₁₀-C₁₃-Isoparaffin) in gleichen Gewichtsanteilen enthalten. Weitere bevorzugte Gewichtsverhältnisse Triethylcitrat/Isopropylmyristat/C₁₀₋₁₃₋Isoparaffin sind (1-1,3) : (0,6-1) : (1-3). Weitere bevorzugte Gewichtsverhältnisse Triethylcitrat/Isopropylmyristat/C₁₀-C₁₃-Isoparaffin sind (1-1,3) : 1 : (1-1,5). Weitere bevorzugte Gewichtsverhältnisse Triethylcitrat/Isopropylmyristat/C₁₀-C₁₃Isoparaffin sind (1-1,3):(0,6-0,9):(2,5-3), insbesondere 1:0,8:3.

In bevorzugten Ölmischungen sind alle drei Öltypen (Triethylcitrat/Isopropylpalmitat/C₁₀-C₁₃-Isoparaffin) in gleichen Gewichtsanteilen enthalten. Weitere bevorzugte Gewichtsverhältnisse Triethylcitrat/Isopropylpalmitat/C₁₀₋₁₃₋Isoparaffin sind (1-1,3) : (0,6-1) : (1-3). Weitere bevorzugte Gewichtsverhältnisse Triethylcitrat/Isopropylpalmitat/C₁₀-C₁₃-Isoparaffin sind (1-1,3) : 1 : (1-1,5). Weitere bevorzugte Gewichtsverhältnisse Triethylcitrat/Isopropylpalmitat/C₁₀-C₁₃-Isoparaffin sind (1-1,3):(0,6-0,9):(2,5-3), insbesondere 1:0,8:3.

In bevorzugten Ölmischungen sind alle drei Öltypen (Triethylcitrat/C₁₂-C₁₅-Alkylbenzoat/C₁₀-C₁₃-Isoparaffin) in gleichen Gewichtsanteilen enthalten. Weitere bevorzugte Gewichtsverhältnisse Triethylcitrat/C₁₂-C₁₅-Alkylbenzoat/C₁₀₋₁₃₋Isoparaffin sind (1-1,3) : (0,6-1) : (1-3). Weitere bevorzugte Gewichtsverhältnisse Triethylcitrat/C₁₂-C₁₅-Alkylbenzoat/C₁₀-C₁₃₋Isoparaffin sind (1-1,3): 1 : (1-1,5). Weitere bevorzugte Gewichtsverhältnisse Triethylcitrat/C₁₂-C₁₅-Alkylbenzoat/C₁₀-C₁₃-Isoparaffin sind (1-1,3):(0,6-0,9):(2,5-3), insbesondere 1:0,8:3.

Es kann erfindungsgemäß außerordentlich bevorzugt sein, Mischungen der vorgenannten Öle einzusetzen, um eine optimale Feinabstimmung der Produkteigenschaften, insbesondere des Rückstandsverhaltens, des Hautgefühls oder der Wirkstofffreisetzung, zu erzielen.

Duft- und Riechstoffe zählen erfindungsgemäß nicht zu den kosmetischen Ölen, die bei der Berechnung des Gewichtsanteils der vorstehend beschriebenen Trägeröle b) berücksichtigt werden. Beispiele für Duft- und Riechstoffverbindungen vom Typ der Ester sind Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Beispiele für Duft- und Riechstoffverbindungen vom Typ der Ether sind Benzylethylether und Ambroxan, Beispiele für Duft- und Riechstoffverbindungen vom Typ der Aldehyde sind die linearen Alkanale mit 8 - 18 C-Atomen, Citral, Citronellal, Citronellyloxy-acetaldehyd, Cyclamenaldehyd, Lilial und Bourgeonal, Beispiele für Duft- und Riechstoffverbindungen vom Typ der Ketone sind die Jonone, alpha-Isomethylionon und Methylcedrylketon, Beispiele für Duft- und Riechstoffverbindungen vom Typ der Alkohole sind Anethol, Citronellol, Eugenol, Geraniol, Linalool, Phenylethylalkohol und Terpineol, Beispiele für Duft- und Riechstoffverbindungen vom Typ der Terpene sind Limonen und Pinen. Beispiele für Duft- und Riechstoffverbindungen sind Pine-, Citrus-, Jasmin-, Patchouly-, Rosen-, Ylang-Ylang-ÖI, Muskateller-Salbeiöl, Kamillenöl, Nelkenöl, Minzöl, Zimtblätteröl, Lindenblütenöl, Wacholderbeeröl, Vetiveröl, Olibanumöl, Galbanumöl, Labdanumöl, Orangenblütenöl, Neroliöl, Orangenschalenöl und Sandelholzöl, weiterhin die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Geraniumöl, Gingergrasöl, Guajakholzöl, Gurjunbalsamöl, Helichrysumöl, Ho-ÖI, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Niaouliöl, Orangenöl, Origanumöl, Palmarosaöl, Patschuliöl, Perubalsamöl, Petitgrainöl, Pfefferöl, Pfefferminzöl, Pimentöl, Pine-ÖI, Rosenöl, Rosmarinöl, Sandelholzöl, Sellerieöl, Spiköl, Sternanisöl, Terpentinöl, Thujaöl, Thymianöl, Verbenaöl, Wacholderbeeröl, Wermutöl, Wintergrünöl, Ysop-ÖI, Zimtöl, Zitronellöl, Zitronenöl und Zypressenöl. Weitere Duft- und Riechstoffverbindungen sind Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadecanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undecalacton, Vanillin, Veratrumaldehyd, Zimtaldehyd, Zimatalkohol, Zimtsäure, Zimtsäureethylester und Zimtsäurebenzylester. Weitere (leichter flüchtige) Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral und Zitronellal.

Bevorzugt werden Mischungen verschiedener Duftstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-ÖI. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Bevorzugte erfindungsgemäße Zusammensetzungen sind dadurch gekennzeichnet, dass mindestens ein Duftstoff in einer Gesamtmenge von 0,1 - 15 Gew.-%, bevorzugt 0,5 - 10 Gew.-%, besonders bevorzugt 1 - 8 Gew.-%, außerordentlich bevorzugt 2 - 7 Gew.-%, weiterhin außerordentlich bevorzugt 3 - 6 Gew.-%, jeweils bezogen auf das Gesamtgewicht der treibmittelfreien Zusammensetzung, enthalten ist.

Weitere erfindungsgemäß bevorzugte Zusammensetzungen sind gekennzeichnet durch einen Gehalt an mindestens einem so genannten "hautkühlenden Wirkstoff'. Unter hautkühlenden Wirkstoffen im Sinne der vorliegenden Anmeldung werden Wirkstoffe verstanden, die bei Applikation auf die Haut infolge Oberflächenanästhesierung und Reizung der kälteempfindlichen Nerven bei Migräne und dergleichen ein angenehmes Kältegefühl erzeugen, auch wenn die behandelten Hautpartien tatsächlich normale bzw. erhöhte Temperatur zeigen.

Bevorzugte hautkühlende Wirkstoffe sind insbesondere Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als hautkühlende Wirkstoffe besonders bevorzugt sind Menthol, Isopulegol, Menthyllactat, Menthoxypropandiol und Menthylpyrrolidoncarbonsäure.

Bevorzugte erfindungsgemäße Zusammensetzungen enthalten mindestens einen hautkühlenden Wirkstoff in Gesamtmengen von 0,01 - 1 Gew.-%, bevorzugt 0,02 - 0,5 Gew.-% und besonders bevorzugt 0,05 - 0,2 Gew.-%, jeweils bezogen auf das Gesamtgewicht der (treibmittelfreien) Zusammensetzung.

Bevorzugte erfindungsgemäße Zusammensetzungen sind dadurch gekennzeichnet, dass mindestens ein verkapselter Wirkstoff enthalten ist. Die Wirkstoffe, die vorteilhafterweise verkapselt sein können, sind insbesondere Duftstoffe, Parfümöle und/oder hautkühlende Wirkstoffe, aber auch andere hautpflegende Wirkstoffe, wie Vitamine, Antioxidantien etc.

Als Kapselmaterial bevorzugt sind wasserlösliche Polymere wie Stärke, physikalisch und/oder chemisch modifizierte Stärken, Cellulosederivate, wie z. B. Carboxymethylcellulose, Methylcellulose, Hydroxyethylcellulose oder Hydroxypropylmethylcellulose, Carragheene, Alginate, Maltodextrine, Dextrine, Pflanzengummen, Pektine, Xanthane, Polyvinylacetat und Polyvinylalkohol, Polyvinylpyrrolidin, Polyamide, Polyester und Homo- und Copolymere aus Monomeren, ausgewählt aus Acrylsäure, Methacrylsäure, Maleinsäure, Fumarsäure, Itaconsäure sowie den Estern und den Salzen dieser Säuren, sowie beliebige Mischungen dieser Polymeren. Bevorzugte Kapselmaterialien sind chemisch modifizierte Stärken, insbesondere Aluminiumstärkeoctenylsuccinat, z. B. das Handelsprodukt Dry Flo Plus von National Starch, oder Natriumstärkeoctenylsuccinat, z. B. das Handelsprodukt Capsul von National Starch, des weiteren Carboxymethylcellulose, Carboxymethylcellulose, Methylcellulose, Hydroxyethylcellulose und Hydroxypropylmethylcellulose, Ethylcellulose, z. B. das Handelsprodukt Tylose H 10 von Clariant, weiterhin Carragheene, Alginate und Maltodextrine, sowie beliebige Mischungen dieser Polymere.

In einer weiteren erfindungsgemäß bevorzugten Ausführungsform enthalten die erfindungsgemäßen Zusammensetzungen 0 bis maximal 5 Gew.-% Ethanol.

Weiterhin können die erfindungsgemäßen Zusammensetzungen zusätzliche Deodorant-Wirkstoffe enthalten. Als Deodorant-Wirkstoffe können antimikrobielle, antibakterielle oder keimhemmende Stoffe, Antioxidantien oder Geruchsadsorbentien (z. B. Zinkricinoleat) eingesetzt werden. Geeignete antimikrobielle, antibakterielle oder keimhemmende Stoffe sind insbesondere Organohalogenverbindungen sowie -halogenide, quartäre Ammoniumverbindungen, eine Reihe von Pflanzenextrakten und Zinkverbindungen. Bevorzugt sind halogenierte Phenolderivate wie z. B. Hexachlorophen oder Irgasan DP 300 (Triclosan, 2,4,4'-Trichlor-2'-hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Auch Benzylalkohol kann als Deodorant-Wirkstoff eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Bevorzugte Deodorant-Wirkstoffe sind langkettige Diole, z. B. 1,2-Alkan-(C₅-C₁₈)-Diole, Glycerinmono(C₈-C₁₈)-Fettsäureester oder, besonders bevorzugt, Glycerinmono-(C₆-C₁₆)-alkylether, insbesondere 2-Ethylhexylglycerinether, die sehr gut haut- und schleimhautverträglich und gegen Corynebakterien wirksam sind, sowie weiterhin Phenoxyethanol, Phenoxyisopropanol (3-Phenoxy-propan-2-ol), Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1-ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethylpropan-1-ol, 2-Allyl-3-phenylpropan-l-ol und 2-n-Pentyl-3-phenylpropan-1-ol.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind z. B. die Salze der Ethylendiamintetraessigsäure oder der Nitrilotriessigsäure sowie die Salze der 1-Hydroxyethan-1,1-diphosphonsäure.

Die Konfektionierung der erfindungsgemäßen Zusammensetzungen, die als Spray appliziert werden, richtet sich vorzugsweise nach den Anforderungen der gewünschten Sprayapplikation. Die erfindungsgemäßen Zusammensetzungen liegen als Suspension vor, das heißt, der schweißhemmende Wirkstoff und gegebenenfalls weitere unlösliche Bestandteile sind in einem flüssigen Träger suspendiert. Ein solches disperses System sollte vor der Anwendung geschüttelt werden. In einer weiteren erfindungsgemäß bevorzugten Ausführungsform sind die erfindungsgemäßen Zusammensetzungen als mit einem Treibmittel versprühbare Suspension konfektioniert. Bevorzugte erfindungsgemäße Zusammensetzungen können z. B. in Pump- oder Quetschspendern abgepackt sein, insbesondere in Mehrkammer-Pump- oder Quetschspendern. Derartige Spender verwenden Luft, insbesondere die Umgebungsluft, als Treibmittel bzw. fördern die erfindungsgemäße Zusammensetzung durch Pumpen.

In einer weiteren bevorzugten Ausführungsform der Erfindung wird die Zusammensetzung mittels eines komprimierten bzw. verflüssigten Treibmittels appliziert.

Alle Mengenangaben beziehen sich, sofern nicht anders angegeben, auf das Gewicht der treibmittelfreien Zusammensetzung.

Die Verpackung in einem Mehrkammerspender bietet besondere technische Vorteile.

Der Mehrkammerspender kann auch so eingesetzt werden, dass eine Kammer mit der erfindungsgemäßen Zusammensetzung befüllt ist, während eine andere Kammer das komprimierte Treibmittel enthalt. Ein derartiger Mehrkammerspender ist beispielsweise eine so genannte Bagin-Can-Verpackung.

Beide Kammern können aber auch so miteinander verbunden, dass die erfindungsgemäße Zusammensetzung in zwei Teilzusammensetzungen aufgetrennt wird, die gleichzeitig aus der Verpackung ausgegeben werden können, beispielsweise aus getrennten Öffnungen oder aus einer einzigen Öffnung.

Weitere bevorzugte erfindungsgemäße Zusammensetzungen sind dadurch gekennzeichnet, dass sie mit mindestens einem Treibmittel in einem geeigneten Druckbehälter verpackt sind.

Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dirhlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-ChCar-1-fluorethan, 2-Chlor-1,1-difluorathen, 1,1,1,3-Tetrafluorethan, Heptafluoro-n-propan, Perfluorethan, Monochlordifluormethan, 1,1-Difluorethan, und zwar sowohl einzeln als auch in Kombination.

Besonders bevorzugt sind Propan, n-Butan, iso-Butan sowie, besonders bevorzugt, Mischungen dieser Treibmittel. Weiterhin bevorzugt sind auch 1,1-Difluorethan, Propan, n-Butan, iso-Butan sowie Mischungen dieser Treibmittel, insbesondere Mischungen aus 1,1-Difluorethan und π-Butan.

Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann. Die Menge der Treibmittel beträgt bevorzugt 1 - 95 Gew.-%, besonders bevorzugt 30 - 90 Gew.-% und außerordentlich bevorzugt 60 - 86 Gew.-%, und weiterhin außerordentlich bevorzugt 70, 72, 74, 76, 78, 82, 64 oder 85 Gew.-%, jeweils bezogen auf das Gesamtgewicht der Zubereitung, bestehend aus der erfindungsgemäßen Suspension und dem Treibmittel

Erfindungsgemäß bevorzugte Zusammensetzungen sind dadurch gekennzeichnet, dass sie als mit einem Treibmittel versprühbare Suspension oder Lösung konfektioniert sind und ein Gewichtsverhältnis von Treibmittel zu treibmittelfreier Zusammensetzung von 20:80 bis 95:5 aufweisen.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der erfindungsgemäßen Suspension. Ein erfindungsgemäß bevorzugter Innenschutzlack ist ein Epoxy-Phenollack, wie er u.a. unter der Bezeichnung Hoba 7407 P erhältlich ist. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.

Die Dosen sind mit einem geeigneten Sprühkopf ausgestattet. Je nach Sprühkopf sind Ausstoßraten, bezogen auf voll gefüllte Dosen, von 0,1 g/s bis 2,0 g/s möglich.

Ein weiterer Gegenstand der vorliegenden Anmeldung ist die Verwendung mindestens eines Alkyl-modifizierten Polyethers der allgemeinen Formel ACTIVATOR-(I) 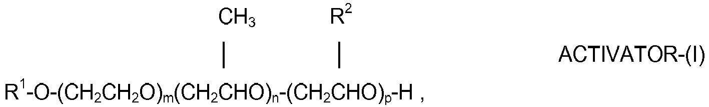 worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10 und p eine rationale Zahl von 1 bis 10 bedeutet, in einer schweißhemmenden Zusammensetzung, enthaltend mindestens einen schweißhemmenden Wirkstoff und 0 - 7 Gew.-%, bevorzugt 0 - 3 Gew.-%, freies Wasser, zur Verbesserung der Schweißreduktion.

Unter "Verbesserung der Schweißreduktion" ist erfindungsgemäß sowohl eine Reduzierung der Schweißmenge als auch eine Beschleunigung der Freisetzung des schweißhemmenden Wirkstoffs aus der erfindungsgemäßen Zusammensetzung zu verstehen.

Ein weiterer Gegenstand der vorliegenden Anmeldung ist die Verwendung mindestens eines Alkyl-modifizierten Polyethers der allgemeinen Formel ACTIVATOR-(I) 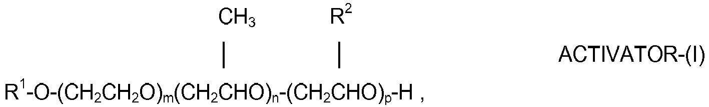 worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10 und p eine rationale Zahl von 1 bis 10 bedeutet, in einer schweißhemmenden Zusammensetzung, enthaltend mindestens einen schweißhemmenden Wirkstoff und 0 - 7 Gew.-%, bevorzugt 0 - 3 Gew.-%, freies Wasser, zur Verbesserung der Schweißreduktion, wobei der Alkyl-modifizierte Polyether in einer Zusammensetzung gemäß einem der Ansprüche 1 - 12 vorliegt.

Ein weiterer Gegenstand der vorliegenden Anmeldung ist die nicht-therapeutische, kosmetische Verwendung einer schweißhemmenden Zusammensetzung gemäß Anspruch 1, 2, 3, 4, 5, 6, 7, 8, 9, 10, 11 oder 12 zur Reduzierung und/oder Regulierung der Transpiration und/oder des Körpergeruchs.

Bezüglich weiterer bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Anmeldung ist ein nicht-therapeutisches, kosmetisches Verfahren zur Reduzierung und/oder Regulierung der Schweißbildung und/oder des Körpergeruchs, bei dem eine erfindungsgemäße oder erfindungsgemäß bevorzugte Zusammensetzung gemäß einem der Ansprüche 1, 2, 3, 4, 5, 6, 7, 8, 9, 10, 11 oder 12 in einer wirksamen Menge auf die Haut, bevorzugt auf die Haut im Achselbereich, aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Die Zusammensetzung einiger bevorzugter erfindungsgemäßer Zusammensetzungen können den folgenden Tabellen entnommen werden (alle Angaben in Gew.-%, bezogen auf das Gesamtgewicht der treibmittelfreien Zusammensetzung):

| | 1 | 2 | 3 | 4 |
|---|---|---|---|---|
| Schweißhemmender Wirkstoff (USP) | 5,0 bis 40 | 10 bis 35 | 15 bis 28 | 23 bis 27 |
| flüssiges Öl als Träger | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)* | 0,01 bis 5 | 0,1 bis 3 | 0,5 bis 2 | 1 bis 1,5 |

| | | | | |
|---|---|---|---|---|
| * worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10, p eine rationale Zahl von 1 bis 10 | | | | |

ist.

| | 5 | 6 | 7 | 8 |
|---|---|---|---|---|
| Schweißhemmendes Aluminiumsalz (USP) | 5,0 bis 40 | 10 bis 35 | 15 bis 28 | 23 bis 27 |
| flüssiges Öl als Träger | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)* | 0,01 bis 5 | 0,1 bis 3 | 0,5 bis 2 | 1 bis 1,5 |

| | | | | |
|---|---|---|---|---|
| * worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10, p eine rationale Zahl von 1 bis 10 | | | | |

ist.

| | 9 | 10 | 11 | 12 |
|---|---|---|---|---|
| Schweißhemmendes Aluminiumsalz (USP) | 5,0 bis 40 | 10 bis 35 | 15 bis 28 | 23 bis 27 |
| flüssiges Öl als Träger | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I) ** | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 13 | 14 | 15 | 16 |
|---|---|---|---|---|
| Schweißhemmendes Aluminiumsalz (USP) | 5,0 bis 40 | 10 bis 35 | 15 bis 28 | 23 bis 27 |
| flüssiges Öl als Träger | 30 bis 95 | 40 bis 93 | 50 bis 90 | 55 bis 85 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |

| | 17 | 18 | 19 | 20 |
|---|---|---|---|---|
| Schweißhemmendes Aluminiumsalz (USP) | 5,0 bis 40 | 10 bis 35 | 15 bis 28 | 23 bis 27 |
| flüchtige cyclische Siliconöle (Cyclomethicone) | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)^{**} | 0,01 bis 5 | 0,1 bis 3 | 0,5 bis 2 | 1 bis 1,5 |

| | | | | |
|---|---|---|---|---|
| ^{**} worin R¹ eine Methylgruppe, R² eine n-Decylgruppe, m eine rationale Zahl von 21 bis 23, n = 0, p eine rationale Zahl von 4 bis 8 ist. | | | | |

| | 21 | 22 | 23 | 24 |
|---|---|---|---|---|
| Schweißhemmendes Aluminiumsalz (USP) | 5,0 bis 40 | 10 bis 35 | 15 bis 28 | 23 bis 27 |
| flüchtige cyclische Siliconöle (Cyclomethicone) | 30 bis 95 | 40 bis 93 | 50 bis 90 | 55 bis 85 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |

| | 25 | 26 | 27 | 28 |
|---|---|---|---|---|
| Schweißhemmendes Aluminiumsalz (USP) | 5,0 bis 40 | 10 bis 35 | 15 bis 28 | 23 bis 27 |
| flüchtige lineare Polydimethylsiloxane mit 2 bis 10 Siloxaneinheiten | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)^{**} | 0,01 bis 5 | 0,1 bis 3 | 0,5 bis 2 | 1 bis 1,5 |

| | | | | |
|---|---|---|---|---|
| ^{**} worin R¹ eine Methylgruppe, R² eine n-Decylgruppe, m eine rationale Zahl von 21 bis 23, n = 0, p eine rationale Zahl von 4 bis 8 ist. | | | | |

| | 29 | 30 | 31 | 32 |
|---|---|---|---|---|
| Schweißhemmendes Aluminiumsalz (USP) | 5,0 bis 40 | 10 bis 35 | 15 bis 28 | 23 bis 27 |
| flüchtige lineare Polydimethylsiloxane mit 2 bis 10 Siloxaneinheiten | 30 bis 95 | 40 bis 93 | 50 bis 90 | 55 bis 85 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |

| | 33 | 34 | 35 | 36 |
|---|---|---|---|---|
| Schweißhemmendes Aluminiumsalz (USP) | 5,0 bis 40 | 10 bis 35 | 15 bis 28 | 23 bis 27 |
| i) Esteröl, ausgewählt aus 2-Ethylhexylpalmitat, Isopropylmyristat und Isopropylpalmitat, und ii) mindestens ein Isoparaffinöl, ausgewählt aus Isodecan, Isoundecan, Isododecan, Isotridecan, Isotetradecan, Isopentadecan, Isohexadecan und Isoeicosan, und iii) 0 - maximal 1 Gew.-% Cyclomethicone | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)** | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 37 | 38 | 39 | 40 |
|---|---|---|---|---|
| Schweißhemmendes Aluminiumsalz (USP) | 5,0 bis 40 | 10 bis 35 | 15 bis 28 | 23 bis 27 |
| i) Esteröl, ausgewählt aus 2-Ethylhexylpalmitat, Isopropylmyristat und Isopropylpalmitat, und ii) mindestens ein Isoparaffinöl, ausgewählt aus Isodecan, Isoundecan, Isododecan, Isotridecan, Isotetradecan, Isopentadecan, Isohexadecan und Isoeicosan, und iii) 0 - maximal 1 Gew.-% Cyclomethicone | 30 bis 95 | 40 bis 93 | 50 bis 90 | 55 bis 85 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |

| | 41 | 42 | 43 | 44 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)^{**} | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 ist. | | | | |

| | 45 | 46 | 47 | 48 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 93 | 50 bis 90 | 55 bis 85 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |

| | 49 | 50 | 51 | 52 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)^{**} | 0,01 bis 5 | 0,1 bis 3 | 0,5 bis 2 | 1 bis 1,5 |
| Cyclomethicone | 0 bis 2 | 0 bis 2 | 0 bis 1 | 0 bis 1 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 ist. | | | | |

| | 53 | 54 | 55 | 56 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 93 | 50 bis 90 | 55 bis 85 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| Cyclomethicone | 0 bis 2 | 0 bis 2 | 0 bis 1 | 0 bis 1 |

| | 57 | 58 | 59 | 60 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 93 | 50 bis 90 | 55 bis 85 |
| freies Wasser | 0 bis 7 | 0 bis 7 | 0 bis 3 | 0 bis 3 |
| ACTIVATOR-(I)^{**} | 0,01 bis 5 | 0,1 bis 3 | 0,5 bis 2 | 1 bis 1,5 |
| Cyclomethicone | 0 bis 2 | 0 bis 2 | 0 bis 1 | 0 bis 1 |
| hydrophobiertes Tonmineral | 0,5 bis 10 | 1 bis 7 | 2 bis 6 | 3 bis 5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 ist. | | | | |

| | 61 | 62 | 63 | 64 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 93 | 50 bis 90 | 55 bis 85 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| Cyclomethicone | 0 bis 2 | 0 bis 2 | 0 bis 1 | 0 bis 1 |
| hydrophobiertes Tonmineral | 0,5 bis 10 | 1 bis 7 | 2 bis 6 | 3 bis 5 |

Die vorstehend aufgeführten erfindungsgemäßen schweißhemmenden Suspensions-Zusammensetzungen (Füllgut) 1 - 64 wurden in Spraydosen aus innen lackiertem Aluminium oder Weißblech (lackiert und unlackiert) abgefüllt und mit einer Isobutan/Butan/Propan-Treibmittel-Mischung im Gewichtsverhältnis Suspension : Treibmittel von 30:70, 25 : 75, 22 : 78, 20 : 80, 18 : 82, 15 : 85 und 13 : 87 beaufschlagt.

Die nachfolgenden Beispiele sollen die Erfindung verdeutlichen, ohne sie hierauf zu beschränken. Alle Mengenangaben sind in Gew.-%.

Die erfindungsgemäßen schweißhemmenden Suspensions-Zusammensetzungen (Füllgut) 1 - 11 und 14 wurden in Spraydosen aus innen lackiertem Aluminium oder Weißblech (lackiert und unlackiert) abgefüllt und mit einer Isobutan/Butan/Propan-Treibmittel-Mischung im Gewichtsverhältnis Suspension : Treibmittel von 30:70, 25 : 75, 22 : 78, 20 : 80, 18 : 82, 15 : 85 und 13 : 87 beaufschlagt; die erfindungsgemäßen Zusammensetzungen 12 und 13 wurden mit einer n-Butan/ 1,1-Difluorethan-Treibmittel-Mischung im Gewichtsverhältnis Suspension : Treibmittel von 45:55, 40:60 und 30:70 beaufschlagt.

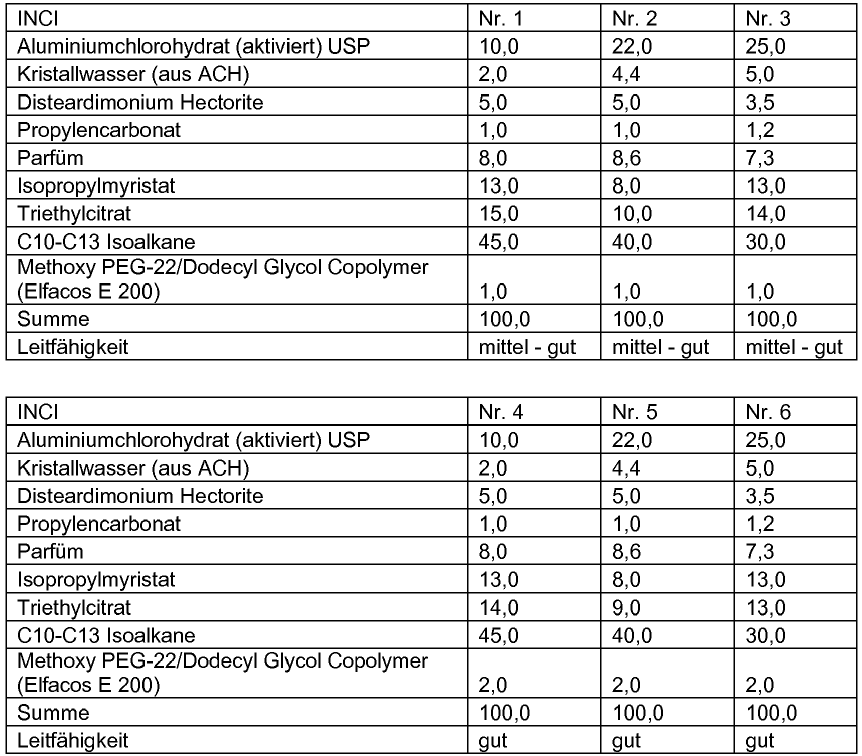

Messung der Freisetzung des schweißhemmenden Wirkstoffs

Zum Nachweis, ob der schweißhemmende Wirkstoff schnell verfügbar ist, wird der zeitliche Verlauf der Leitfähigkeit der Suspensionen aus einem definierten Film in einer bestimmten Menge entionisierten Wassers gemessen.

Der Wert für die am Ende des Versuchs erreichte Leitfähigkeit liegt für die erfindungsgemäßen Zusammensetzungen bei 25 - 160 Mikrosiemens pro Zentimeter [µS/cm].

Die Zusammensetzungen ohne Methoxy PEG-22/Dodecyl Glycol Copolymer wiesen eine finale Leitfähigkeit von maximal 15 Mikrosiemens pro Zentimeter [µS/cm] auf.

Die erfindungsgemäßen und die Vergleichs-Zusammensetzungen Nr. 1 bis Nr. 6 wurden auf die Haut im Achselbereich aufgetragen.

Bei den erfindungsgemäßen Zusammensetzungen wurde eine schneller einsetzende schweißhemmende Wirkung beobachtet.

In den vorstehenden Tabellen ist eine qualitative Beurteilung der getesteten Zusammensetzungen vermerkt, mit einer Notenskala von 1 - 5 mit 1 = sehr gut, 5 = schlecht. In diese Beurteilung geht nicht nur der erreichte Endwert der Leitfähigkeit ein, sondern auch die Steigung der zeitlichen Veränderung der Leitfähigkeit zu Beginn des Versuchs. Eine starke Steigung wird als gleichbedeutend mit einer schnellen Freisetzung des schweißhemmenden Wirkstoffs interpretiert.

Weitere erfindungsgemäße Formulierungsbeispiele

### (Gewichtsverhältnis Füllgut zu Treibmittel wie bei den vorstehenden Beispielen):

| Nr. | 7 | 8 | 9 | 10 | 11 | 12 | 13 | 14 |
|---|---|---|---|---|---|---|---|---|
| Parfum | 5,0 | 5,0 | 7,00 | 5,0 | 5,0 | 5,0 | 7,0 | 5,0 |
| Aluminumchlorohydrat (aktiviert), inclusive Kristallwasser | 32,0 | 28,0 | 26,0 | 35,0 | 32,0 | 32,0 | 30,0 | 28,0 |
| verkapseltes 2-Benzylheptanol, Phenoxyethanol (Na-Stärkeoctenylsuccinat, Mannitol, Silica) | 2,0 | -- | -- | 2,0 | 1,0 | -- | 1,0 | -- |
| Methoxy PEG-22/ Dodecyl Glycol Copolymer (Elfacos E 200) | 1,0 | 1,0 | 2,0 | 2,0 | 2,0 | 1,5 | 1,5 | 1,5 |
| Isopropylpalmitat | -- | -- | - | - | - | 5,0 | - | -- |
| C10-13 Isoparaffin | 25,0 | 30,0 | 25,0 | - | - | - | - | -- |
| C12-15-Alkylbenzoat | -- | -- | - | 5,0 | 5,0 | -- | -- | -- |
| Cosmacol PLG | - | - | - | - | 5,0 | 5,0 | 5,0 | - |
| Disteardimonium Hectorite | 4,5 | 3,9 | 4,0 | 3,8 | 5,0 | 4,5 | - | 3,9 |
| Propylencarbonat | 1,5 | 1,3 | 1,0 | 1,3 | 1,70 | 1,5 | - | 1,3 |
| Silica | - | - | - | - | - | 0,8 | 0,8 | - |
| Silica Dimethyl Silylate | - | - | - | - | - | 1,0 | 1,0 | - |
| Tocopherylacetat | 0,2 | - | 0,5 | 0,1 | -- | -- | -- | -- |
| Cyclopentasiloxan | -- | -- | -- | ad 100 | ad 100 | ad 100 | ad 100 | ad 100 |
| Isopropylmyristat | - | ad 100 | - | - | - | - | - | 5,0 |
| 2-Ethylhexylpalmitat | ad 100 | - | ad 100 | - | - | - | - | - |

Cosmacol PLG (INCI: DI-C12-13 ALKYL TARTRATE, TRI-C12-13 ALKYL CITRATE, SILICA)

## Patentansprüche

1. Schweißhemmende Zusammensetzung zur persönlichen Körperpflege, konfektioniert als treibmittelfrei oder mit einem Treibmittel versprühbare Suspension oder Lösung, enthaltend
a) mindestens einen schweißhemmenden Wirkstoff,
b) mindestens ein unter Normalbedingungen flüssiges ÖI als Träger,
c) 0 - 7 Gew.-%, bevorzugt 0 - 3 Gew.-%, freies Wasser, bezogen auf das Gewicht der treibmittelfreien Zusammensetzung,
d) mindestens einen Alkyl-modifizierten Polyether der allgemeinen Formel ACTIVATOR-(I) 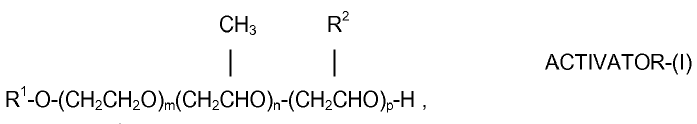
worin R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet.

2. Zusammensetzung gemäß Anspruch 1, **dadurch gekennzeichnet, dass** der Alkyl-modifizierte Polyether d) ausgewählt ist aus Verbindungen der allgemeinen Formel ACTIVATOR-(I), worin R¹ ausgewählt ist aus einer Methylgruppe, einer Ethylgruppe, einer n-Propylgruppe und einer 1-Methylethylgruppe, bevorzugt einer Methylgruppe,
R² ausgewählt ist aus einer n-Octyl-, 2-Ethylhexyl-, n-Nonyl-, n-Decyl-, 2-Ethyloctyl-, n-Undecyl-, n-Dodecyl-, 2-Ethyldecyl-, n-Tridecyl-, Myristyl-, n-Pentadecyl-, Cetyl-, Palmityl-, Stearyl-, Elaidyl-, Arachidyl-, Behenyl- oder Cocyl-Gruppe, bevorzugt einer n-Decyl-Gruppe, m eine Zahl von 12 - 30, bevorzugt 22, darstellt,
n eine rationale Zahl von 0 - 8, bevorzugt 0 - 4, besonders bevorzugt 0, darstellt,
p eine rationale Zahl von 1 - 9, bevorzugt 4 - 8, besonders bevorzugt 5 - 7, darstellt.

3. Zusammensetzung gemäß Anspruch 1 oder 2, **dadurch gekennzeichnet, dass** der Alkyl-modifizierte Polyether d) der allgemeinen Formel ACTIVATOR-(I) einen HLB-Wert im Bereich von 5 - 7, bevorzugt 6 - 6,8, besonders bevorzugt 6,2 - 6,5, aufweist.

4. Zusammensetzung gemäß einem der vorstehenden Ansprüche, **dadurch gekennzeichnet, dass** mindestens ein Alkyl-modifizierter Polyether der allgemeinen Formel ACTIVATOR-(I) enthalten ist mit einem HLB-Wert im Bereich von 5 - 7, bevorzugt 6 - 6,8, besonders bevorzugt 6,2 - 6,5, und mit R¹ = Methyl, R² = n-Decyl, m = 22, n = 0 und p = 4 - 5.

5. Zusammensetzung gemäß Anspruch 1, 2, 3 oder 4, **dadurch gekennzeichnet, dass** mindestens ein Alkyl-modifizierter Polyether der allgemeinen Formel ACTIVATOR-(I) enthalten ist mit einem HLB-Wert im Bereich von 5 - 7, bevorzugt 6 - 6,8, besonders bevorzugt 6,2 - 6,5, und mit R¹ = Methyl, R² = n-Decyl, m = 22, n = 0 und p = 7 - 8.

6. Zusammensetzung gemäß Anspruch 1, 2, 3, 4 oder 5, **dadurch gekennzeichnet, dass** der Alkyl-modifizierte Polyether d) der allgemeinen Formel ACTIVATOR-(I) ausgewählt ist aus Verbindungen mit der INCI-Bezeichnung Methoxy PEG-22/Dodecyl Glycol Copolymer.

7. Zusammensetzung gemäß einem der vorstehenden Ansprüche, **dadurch gekennzeichnet, dass** das mindestens eine unter Normalbedingungen flüssige Träger-ÖI b) ausgewählt ist aus flüchtigen cyclischen oder linearen Siliconölen, nichtflüchtigen höhermolekularen linearen Dimethylpolysiloxanen, Estern von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Benzoesäureestern von linearen oder verzweigten C₈₋₂₂-Alkanolen, den C₈-C₂₂-Fettalkoholestern einwertiger oder mehrwertiger C₂-C₇-Hydroxycarbonsäuren, den Anlagerungsprodukten von Ethylenoxid und/oder Propylenoxid an ein- oder mehrwertige C₃₋₂₀-Alkanole, flüssigen Paraffinölen, Isoparaffinölen, insbesondere Isodecan, Isoundecan, Isododecan, Isotridecan, Isotetradecan, Isopentadecan, Isohexadecan und Isoeicosan, synthetischen Kohlenwasserstoffen, wie Polyisobuten oder Polydecene, und alicyclischen Kohlenwasserstoffen, verzweigten gesättigten oder ungesättigten Fettalkoholen mit 6 - 30 Kohlenstoffatomen, Mischungen aus Guerbetalkoholen und Guerbetalkoholestern, den symmetrischen, unsymmetrischen oder cyclischen Estern der Kohlensäure mit Fettalkoholen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Di-n-alkylethern mit insgesamt 12 bis 36, insbesondere 12 bis 24 C-Atomen, sowie Mischungen der vorgenannten Öle.

8. Zusammensetzung gemäß einem der vorstehenden Ansprüche, **dadurch gekennzeichnet, dass** das mindestens eine unter Normalbedingungen flüssige Träger-ÖI b) mindestens ein Isoparaffinöl, insbesondere Isodecan, Isoundecan, Isododecan, Isotridecan, Isotetradecan, Isopentadecan, Isohexadecan und Isoeicosan, umfasst.

9. Zusammensetzung gemäß einem der Ansprüche 1, 2, 3, 4, 5, 6, 7 oder 8, **dadurch gekennzeichnet, dass** 0 - maximal 1 Gew.-% Cyclomethicone enthalten ist.

10. Zusammensetzung gemäß einem der Ansprüche 1, 2, 3, 4, 5, 6, 7, 8 oder 9, **dadurch gekennzeichnet, dass** das unter Normalbedingungen flüssige Träger-ÖI b) aus einer Mischung aus b)i) einem Esteröl, ausgewählt aus 2-Ethylhexylpalmitat, Isopropylmyristat und Isopropylpalmitat, und b)ii) mindestens einem Isoparaffinöl, ausgewählt aus Isodecan, Isoundecan, Isododecan, Isotridecan, Isotetradecan, Isopentadecan, Isohexadecan und Isoeicosan, und b)iii) 0 - maximal 1 Gew.-% Cyclomethicone besteht.

11. Zusammensetzung gemäß einem der vorstehenden Ansprüche, **dadurch gekennzeichnet, dass** sie als mit einem Treibmittel versprühbare Suspension oder Lösung konfektioniert ist.

12. Zusammensetzung gemäß Anspruch 11, **gekennzeichnet durch** ein Gewichtsverhältnis von Treibmittel zu treibmittelfreier Zusammensetzung von 20:80 bis 95:5.

13. Nicht-therapeutisches, kosmetisches Verfahren zur Reduzierung und/oder Regulierung der Schweißbildung und/oder des Körpergeruchs, bei dem eine erfindungsgemäße oder erfindungsgemäß bevorzugte Zusammensetzung gemäß einem der Ansprüche 1, 2, 3, 4, 5, 6, 7, 8, 9, 10, 11 oder 12 in einer wirksamen Menge auf die Haut, bevorzugt auf die Haut im Achselbereich, aufgetragen wird.

14. Verwendung mindestens eines Alkyl-modifizierten Polyethers der allgemeinen Formel ACTIVATOR-(I) 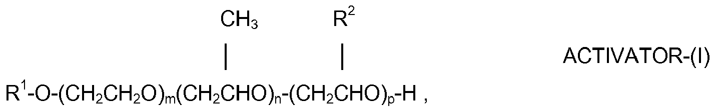
worin R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet, in einer schweißhemmenden Zusammensetzung, enthaltend mindestens einen schweißhemmenden Wirkstoff und 0 - 7 Gew.-%, bevorzugt 0 - 3 Gew.-%, freies Wasser, zur Verbesserung der Schweißreduktion.

15. Verwendung gemäß Anspruch 14, **dadurch gekennzeichnet, dass** der Alkyl-modifizierte Polyether der allgemeinen Formel ACTIVATOR-(I) in einer Zusammensetzung gemäß einem der Ansprüche 1 - 12 vorliegt.

## Claims

1. An antiperspirant composition for personal body care, made up as a suspension or solution which is sprayable with or without a propellant, containing
a) at least one active antiperspirant substance,
b) at least one oil which is liquid under normal conditions as carrier,
c) 0 - 7 wt.%, preferably 0 - 3 wt.%, of free water, based on the weight of the propellant-free composition,
d) at least one alkyl-modified polyether of the general formula ACTIVATOR-(I) 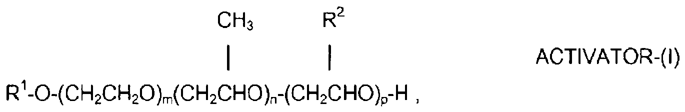
wherein R¹ signifies an aliphatic hydrocarbon residue with 1 to 3 C atoms,
R² signifies an aliphatic hydrocarbon residue with 8 to 30 C atoms,
m is a rational number from 10 to 50,
n is a rational number from 0 to 10,
p is a rational number from 1 to 10.

2. The composition according to claim 1, wherein the alkyl-modified polyether d) is selected from compounds of the general formula ACTIVATOR-(I), wherein
R¹ is selected from a methyl group, an ethyl group, an n-propyl group and a 1-methylethyl group, preferably a methyl group,
R² is selected from an n-octyl, 2-ethylhexyl, n-nonyl, n-decyl, 2-ethyloctyl, n-undecyl, n-dodecyl, 2-ethyldecyl, n-tridecyl, myristyl, n-pentadecyl, cetyl, palmityl, stearyl, elaidyl, arachidyl, behenyl or cocyl group, preferably an n-decyl group,
m represents a number from 12 - 30, preferably 22,
n represents a rational number from 0 - 8, preferably 0 - 4, particularly preferably 0,
p represents a rational number from 1 - 9, preferably 4 - 8, particularly preferably 5-7.

3. The composition according to claim 1 or claim 2, wherein the alkyl-modified polyether d) of the general formula ACTIVATOR-(I) has an HLB value in the range of 5 - 7, preferably 6 - 6.8, particularly preferably 6.2 - 6.5.

4. The composition according to one of the preceding claims, wherein at least one alkyl-modified polyether of the general formula ACTIVATOR-(I) is contained with an HLB value in the range of 5 - 7, preferably 6 - 6.8, particularly preferably 6.2 - 6.5, and with R¹ = methyl, R² = n-decyl, m = 22, n = 0 and p = 4 - 5.

5. The composition according to claim 1, 2, 3 or 4, wherein at least one alkyl-modified polyether of the general formula ACTIVATOR-(I) is contained with an HLB value in the range of 5 - 7, preferably 6 - 6.8, particularly preferably 6.2 - 6.5, and with R¹ = methyl, R² = n-decyl, m = 22, n = 0 and p = 7 - 8.

6. The composition according to claim 1, 2, 3, 4 or 5, wherein the alkyl-modified polyether d) of the general formula ACTIVATOR-(I) is selected from compounds with the INCI name Methoxy PEG-22/Dodecyl Glycol Copolymer.

7. The composition according to one of the preceding claims, wherein the at least one carrier oil b) which is liquid under normal conditions is selected from volatile cyclic or linear silicone oils, non-volatile higher molecular-weight linear dimethyl polysiloxanes, esters of linear or branched saturated or unsaturated fatty alcohols having 2 - 30 carbon atoms with linear or branched saturated or unsaturated fatty acids having 2 - 30 carbon atoms, which may be hydroxylated, benzoic acid esters of linear or branched C₈-₂₂ alkanols, C₈-C₂₂ fatty alcohol esters of monohydric or polyhydric C₂-C₇ hydroxycarboxylic acids, the addition products of ethylene oxide and/or propylene oxide to monohydric or polyhydric C₃₋₂₀ alkanols, liquid paraffin oils, isoparaffin oils, in particular isodecane, isoundecane, isododecane, isotridecane, isotetradecane, isopentadecane, isohexadecane and isoeicosane, synthetic hydrocarbons, such as polyisobutene or polydecene, and alicyclic hydrocarbons, branched saturated or unsaturated fatty alcohols with 6 - 30 carbon atoms, mixtures of Guerbet alcohols and Guerbet alcohol esters, the symmetrical, asymmetrical or cyclic esters of carbonic acid with fatty alcohols, triglycerides of linear or branched, saturated or unsaturated, optionally hydroxylated C₈₋₃₀ fatty acids, dicarboxylic acid esters of linear or branched C₂-C₁₀ alkanols, di-n-alkyl ethers with a total of 12 to 36, in particular 12 to 24 C atoms, and mixtures of the aforementioned oils.

8. The composition according to one of the preceding claims, wherein the at least one carrier oil b) which is liquid under normal conditions comprises at least one isoparaffin oil, in particular isodecane, isoundecane, isododecane, isotridecane, isotetradecane, isopentadecane, isohexadecane and isoeicosane.

9. The composition according to any one of claims 1, 2, 3, 4, 5, 6, 7 or 8, wherein 0 to no more than 1 wt.% of cyclomethicones is contained.

10. The composition according to one of claims 1, 2, 3, 4, 5, 6, 7, 8 or 9, wherein the carrier oil b) which is liquid under normal conditions consists of a mixture of b)i) an ester oil, selected from 2-ethylhexyl palmitate, isopropyl myristate and isopropyl palmitate, and b)ii) at least one isoparaffin oil, selected from isodecane, isoundecane, isododecane, isotridecane, isotetradecane, isopentadecane, isohexadecane and isoeicosane, and b)iii) 0 to no more than 1 wt.% of cyclomethicone.

11. The composition according to any one of the preceding claims, wherein it is formulated as a suspension or solution sprayable with a propellant.

12. The composition according to claim 11, **characterized by** a weight ratio of propellant to propellant-free composition of 20:80 to 95:5.

13. A non-therapeutic, cosmetic method of reducing and/or regulating sweat formation and/or body odor, in which a composition according to the invention, or which is preferred according to the invention, according to one of claims 1, 2, 3, 4, 5, 6, 7, 8, 9, 10, 11 or 12 is applied onto the skin in an effective quantity, preferably onto the skin in the arm pit area.

14. Use of at least one alkyl-modified polyether of the general formula ACTIVATOR-(I) 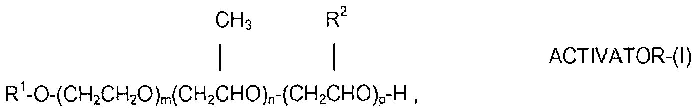
wherein R¹ signifies an aliphatic hydrocarbon residue with 1 to 3 C atoms,
R² signifies an aliphatic hydrocarbon residue with 8 to 30 C atoms,
m is a rational number from 10 to 50,
n is a rational number from 0 to 10,
p is a rational number from 1 to 10,
in an antiperspirant composition containing at least one active antiperspirant substance and 0 - 7 wt.%, preferably 0 - 3 wt.%, of free water for improving sweat reduction.

15. Use according to claim 14, wherein the alkyl-modified polyether of the general formula ACTIVATOR-(I) is present in a composition according to one of claims 1-12.

## Revendications

1. Composition antiperspirante pour soins corporels personnels, confectionnée sous la forme d'une suspension ou une solution pulvérisable avec ou sans agent propulseur,
a) au moins un agent anti-transpirant,
b) au moins une huile liquide dans des conditions normales servant de support,
c) 0 à 7% en poids, de préférence 0 à 3% en poids, d'eau libre, par rapport au poids de la composition sans agent propulseur,
d) au moins un polyéther alkyl-modifié de la formule générale ACTIVATEUR-(I) 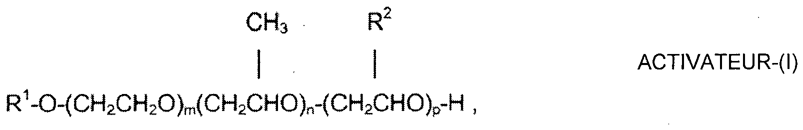
dans laquelle
R¹ est un radical hydrocarboné aliphatique ayant 1 à 3 atomes de carbone,
R² est un radical hydrocarboné aliphatique ayant 8 à 30 atomes de carbone,
m est un nombre rationnel de 10 à 50,
n est un nombre rationnel de 0 à 10,
p est un nombre rationnel de 1 à 10.

2. Composition selon la revendication 1, **caractérisée en ce que** le polyéther alkyle-modifié d) est choisi parmi les composés de la formule générale ACTIVATEUR-(I), dans laquelle R¹ est choisi parmi un groupe méthyle, un groupe éthyle, un groupe n-propyle et un groupe 1-méthyléthyle, de préférence un groupe méthyle,
R² est choisi parmi les groupes n-octyle, 2-éthylhexyle, n-nonyle, n-décyle, 2-éthyloctyle, n-undécyle, n-dodécyle, 2-éthyldecyl-, n-tridécyl-, myristyle, n-pentadécyle, cétyle, palmityle, stéaryle, élaidyle, arachidyle, béhényle ou cocyle, de préférence n-décyle,
m est un nombre de 12 à 30, de préférence 22,
n est un nombre rationnel de 0 à 8, de préférence de 0 à 4, de manière particulièrement préférée 0,
p est un nombre rationnel de 1 à 9, de préférence de 4 à 8, de manière particulièrement préférée de 5 à 7.

3. Composition selon la revendication 1 ou 2, **caractérisée en ce que** le polyéther alkyle-modifié d) de la formule générale ACTIVATEUR-(I) a une valeur HLB dans la gamme de 5 à 7, de préférence de 6 à 6,8, de manière particulièrement préférée de 6,2 à 6,5.

4. Composition selon l'une des revendications précédentes, **caractérisée en ce qu'**elle contient au moins un polyéther alkyle-modifié de la formule générale ACTIVATEUR-(I) ayant une valeur HLB dans la gamme de 5 à 7, de préférence de 6 à 6,8, de manière particulièrement préférée de 6,2 à 6,5, et avec R¹ = méthyle, R² = n-décyle, m = 22, n = 0 et p = 4 ou 5.

5. Composition selon la revendication 1, 2, 3 ou 4, **caractérisée en ce qu'**elle contient au moins un polyéther alkyl-modifié de la formule générale ACTIVATEUR-(I) ayant une valeur HLB dans la gamme de 5 à 7, de préférence 6 de à 6, 8, de manière particulièrement préférée de 6,2 à 6,5, et avec R¹ = méthyle, R² = n-décyle, m = 22, n = 0 et p = 7 ou 8.

6. Composition selon la revendication 1, 2, 3, 4 ou 5, **caractérisée en ce que** le polyéther alkyle-modifié d) de la formule générale ACTIVATEUR-(I) est choisi parmi les composés ayant la dénomination INCI «Methoxy PEG-22/Dodecyl Glycol Copolymer».

7. Composition selon l'une des revendications précédentes, **caractérisée en ce que** l'au moins une huile-support b), liquide dans des conditions normales, est choisie parmi les d'huiles de silicone cycliques ou linéaires volatiles, les diméthylpolysiloxanes linéaires de poids moléculaires élevés non volatiles, les esters d'alcools gras saturés ou insaturés, linéaires ou ramifiés ayant 2 à 30 atomes de carbone, avec des acides gras saturés ou insaturés, linéaires ou ramifiés ayant 2 à 30 atomes de carbone, qui peuvent être hydroxylés, les esters d'acide benzoïque d'alcanols en C₈ à C₂₂ linéaires ou ramifiés, les esters d'alcools gras en C₈ à C₂₂ et d'acides hydroxy-carboxyliques en C₂ à C₇ mono- ou polyvalent, les produits d'addition d'oxyde d'éthylène et/ou d'oxyde de propylène à des alcanols en C₃ à C₂₀ mono-ou polyvalents, les huiles de paraffine liquides, les huiles isoparaffiniques, en particulier l'isododécane, l'isoundécane, l'isododécane, l'isotridécane, l'isotetradécane, l'isopentadecane, l'isohexadécane et l'isoeicosane, les hydrocarbures synthétiques, tels que le polyisobutène, ou les polydécènes, et les hydrocarbures alicycliques, les alcools gras ramifiés, saturés ou insaturés ayant 6 à 30 atomes de carbone, des mélanges d'alcools de Guerbet et d'esters d'alcools de Guerbet, les esters symétriques, asymétriques ou cycliques de l'acide carbonique avec des alcools gras, les triglycérides d'acides gras en C₈ à C₃₀ linéaires ou ramifiés, saturés ou insaturés, éventuellement hydroxylés, les esters d'acides dicarboxyliques d'alcanols en C₂ à C₁₀ linéaires ou ramifiés, les di-n-alkyléthers ayant au total de 12 à 36, en particulier de 12 à 24 atomes de carbone, et des mélanges de des huiles susmentionnées.

8. Composition selon l'une des revendications précédentes, **caractérisée en ce que** l'au moins une huile-support b), liquide dans des conditions normales, contient au moins une huile isoparaffinique, en particulier l'isodécane, l'isoundécane, l'isododécane, l'isotridécane, l'isotétradecane, l'isopentadécane, l'isohexadécane et l'isoeicosane.

9. Composition selon l'une des revendications 1, 2, 3, 4, 5, 6, 7 ou 8, **caractérisée en ce qu'**elle contient de 0 à 1% en poids maximum de cyclométhicone.

10. Composition selon l'une des revendications 1, 2, 3, 4, 5, 6, 7, 8 ou 9, **caractérisée en ce que** le l'huile-support b), liquide dans des conditions normales, est constituée d'un mélange de b)i) une huile d'ester choisie parmi le palmitate de 2-éthylhexyle, le myristate d'isopropyle et le palmitate d'isopropyle, et b) ii) d'au moins une huile isoparaffinique choisie parmi l'isodécane, l'isoundécane, l'isododécane, l'isotridécane, l'isotétradecane, l'isopentadécane, l'isohexadécane et l'isoeicosane, et b)iii) de 0 à 1% en poids maximum de cyclométhicone.

11. Composition selon l'une des revendications précédentes, **caractérisée en ce qu'**elle est formulée sous forme de solution ou de suspension pulvérisable avec un agent propulseur.

12. Composition selon la revendication 11, **caractérisée par** un rapport en poids agent propulseur sur composition sans propulseur de 20:80 à 95:5.

13. Procédé cosmétique non thérapeutique de réduction et/ou de régulation de la transpiration et/ou l'odeur corporelle, dans lequel une composition de l'invention ou préférée de l'invention selon l'une des revendications 1, 2, 3, 4, 5, 6, 7, 8, 9, 10, 11 ou 12 est appliquée dans une quantité efficace sur la peau, de préférence sur la peau dans la région des aisselles.

14. Utilisation d'au moins un polyéther alkyle-modifié de la formule générale ACTIVATEUR-(I) 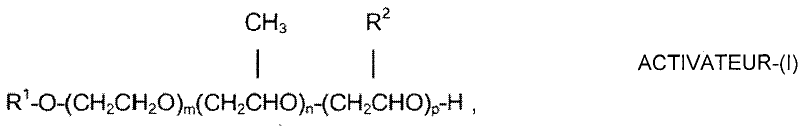 dans laquelle
R¹ est un radical hydrocarboné aliphatique ayant 1 à 3 atomes de carbone,
R² est un radical hydrocarboné aliphatique ayant 8 à 30 atomes de carbone,
m est un nombre rationnel de 10 à 50,
n est un nombre rationnel de 0 à 10,
p est un nombre rationnel de 1 à 10
dans une composition antiperspirante contenant au moins un agent antiperspirant et 0 à 7% en poids, de préférence 0 à 3% en poids, d'eau libre pour améliorer la réduction de la transpiration.

15. Utilisation selon la revendication 14, **caractérisée en ce que** le polyéther alkyle-modifié de la formule générale ACTIVATEUR-(I) est présent dans une composition selon l'une des revendications 1 à 12.
